(19) Europäisches Patentamt
European Patent Office
Office européen des brevets

(11) EP 0 771 815 A1

(12) **EUROPEAN PATENT APPLICATION**

(43) Date of publication:
**07.05.1997 Bulletin 1997/19**

(51) Int. Cl.[6]: **C07H 3/06**, C07H 5/08, C07H 11/04, A61K 31/70

(21) Application number: **96117513.0**

(22) Date of filing: **31.10.1996**

(84) Designated Contracting States:
**CH DE FR GB IT LI**

(30) Priority: **31.10.1995 JP 283425/95**
**22.10.1996 JP 279362/96**

(71) Applicant: **SANWA KAGAKU KENKYUSHO CO., LTD.**
**Higashi-ku Nagoya-shi Aichi-ken 461 (JP)**

(72) Inventors:
- **Usui, Toshinao**
  **Nagoya, Aichi-ken, 461 (JP)**
- **Igami, Takao**
  **Nagoya, Aichi-ken, 461 (JP)**
- **Kakigami, Takuji**
  **Nagoya, Aichi-ken, 461 (JP)**
- **Hamashima, Hitoshi**
  **Nagoya, Aichi-ken, 461 (JP)**
- **Jomori, Takahito**
  **Nagoya, Aichi-ken, 461 (JP)**
- **Tashita, Akira**
  **Nagoya, Aichi-ken, 461 (JP)**
- **Ishiwatari, Yoshiro**
  **Nagoya, Aichi-ken, 461 (JP)**
- **Yokochi, Shoji**
  **Nagoya, Aichi-ken, 461 (JP)**
- **Mitani, Takahiko**
  **Nagoya, Aichi-ken, 461 (JP)**
- **Suzuki, Yasuo**
  **Shizuoka-shi, Shizuoka-ken, 420 (JP)**
- **Hasegawa, Akira**
  **Gifu-shi, Gifu-ken, 501-31 (JP)**

(74) Representative: **VOSSIUS & PARTNER**
**Siebertstrasse 4**
**81675 München (DE)**

(54) **Sulfated and phosphated saccharide derivatives, process for the preparation of the same and use thereof**

(57) There are described sulfate or phosphate saccharide derivatives of the Formula

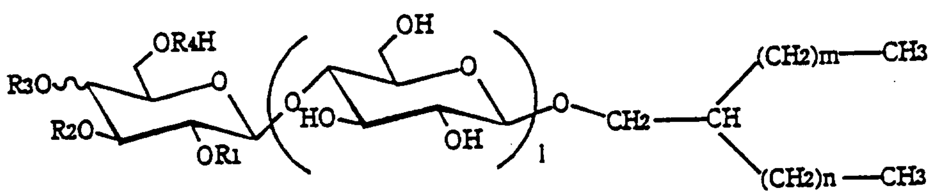

(I)

wherein $R_1$ is hydrogen atom or a residue of sulfate, phosphate or L-fucose; $R_2$, $R_3$ and $R_4$ are hydrogen atom or a residue of sulfate or phosphate, respectively; l is an integer of 0 or 1; m is an integer of 0 - 15; and n is an integer of 0 - 21,
or pharmaceutically acceptable salts thereof, a process for the preparation of the derivatives and salts as well as use thereof, as an anti-inflammatory agent.

EP 0 771 815 A1

## Description

The present invention relates to novel sulfated and phosphated saccharide derivatives, a process for the preparation of the same and the use thereof as well as an anti-inflammatory agent containing the derivative as an effective ingredient thereof.

In recent years, many adhesion molecules have been identified which concern direct cell contact between cells per se or cell and exo-cellular matrix. These adhesion molecules are classified into groups of selectin family, immunoglobulin family, integrin family, CD44 and the like in view of those structural characteristics, and elucidation of various functions thereof have energetically been made. Many of cell adhesion molecules belonging to the selectin family concern immunophlogistic reactions.

At the present time, 3 type cell adhesion molecules (L-selectin, E-selectin and P-selectin) have been known as those belonging to the selectin family. Among them, L-selectin is expressed on lymphocytes, neutrocytes and monocytes, and it has been considered as concerning to homing of the lymphocytes and adhesion of inflamed region to vascular endothelium cells. E-selectin is a protein to be expressed on inflammatory vascular endothelium cells by stimulation of inflammatory cytokine, and mediates the cell adhesion of neutrocytes, monocytes and the like. P-selectin is expressed on activated vascular endothelium cells and activated platelets, and mediates cell adhesion between platelets and leukocytes or vascular endothelium cells and leukocytes.It become clear that these selectins concern rolling on the surface of vascular endothelium cells of the leukocyte rather than those powerful cell adhesing action thereto, which rolling occurs prior to the cell adhesion ["J. IMMUNOL.", Vol. 153, page 3917 (1994)].

Recently, saccharide ligands recognizing these selectins have been elucidated on the molecular level ["NATURE", Vol. 367, page 532 (1994); and "BIOCHEMISTRY", Vol. 33, page 1332 (1994)]. Particularly, it has been found that Sialyl Lewis X and Sialyl Lewis A is a common ligand of E-, L- and P-selectins ["SCIENCE", Vol. 250, page 1130 (1990); "SCIENCE", Vol. 250, page 1132 (1990); "PROC. NATL. ACAD. SCI. USA", Vol. 88, page 6224 (1991); "BIOCHEM, BIOPHY. RES. COM.", Vol. 179, page 713 (1991) and "J. BIOL. CHEM.", Vol. 269, page 19663 (1994)], and various derivatives thereof have been synthesized ["PROC. NATL. ACAD. SCI. USA", Vol. 88, page 10372 (1991); "CARBOHYDR. RES.", Vol. 229. page c1 (1992); "CARBOHYDR. RES.", Vol. 257, page 67 (1994) and "BIOSCI. BIOTECH. BIOCHEM." Vol. 59, page 1091 (1995)]. Further, it has been found that a sulfated saccharide chain as in that of sulfatides and a phosphated poly alcohol strongly bind to selectins and more particularly to P- and L-selectins and thus such a report has been issued that such compounds may show anti-inflammatory action ["CELL". Vol. 67, page 35 (1991); "BIOCHEM.BIOPHY. RES. COM.", Vol. 181, page 1223 (1991); "BIOCHEM. BIOPSY. RES. COM.", Vol. 190, page 426 (1993) and "INT. IMMUNOL.", Vol. 7, page 1107 (1995)].

On the other hand, an acute lung damage model using cobra venom factor (CVF) has been established as selectin depending inflammation model "J. CLIN. INVEST.", Vol. 88, page 1396 (1991); "J. CLIN. INVEST.", Vol. 90, page 1600 (1992) and "J. IMMUNOL.", Vol. 151. page 6410 (1993)].

Recently, reports have been issued as on screening using anti-selectin antibody ["J. IMMUNOL." Vol. 152, page 832 (1994)], Sialyl Lewis X ["NATURE", Vol. 364, page 149 (1993) and "J. EXP. MED.", Vol. 178, page 623 (1993)]or sulfatide ["INT. IMMUNOL.", Vol. 7, page 1107 (1995)] and the like, and thus it has been recognized that inhibition of cell adhesion due to selectin shows an inflammatory effect in various inflammatory reactions such as ischemia reperfusion disease, asthma, skin inflammation, lung injury due to activated complements, a shock caused by bleeding or external wound and rheumatism.

As sulfatide analogues, P. A. Ward et al. have reported that sulfatide and synthetic sulfated galactose derivatives (containing 2-tetradesyl hexadesyl β-D-galactopyranoside 3-sulfate) show inflammation inhibition effect, in case of using 2 type lung injury models, namely an inflammatory model caused activation of whole body complements by administration of CVF and another inflammatory model administrated IgG immunocomplex ["INT. IMMUNOL.", Vol. 7, page 1107 (1995)]. Further, Y. Suzuki et al. have confirmed through in vitro tests using a plastic plate that natural and chemically synthesized sulfated glycolipids [containing 2-tetradesyl hexadesylβ -D-galactopyranoside 3-sulfate and 2-tetradesyl hexadesyl 0-β -D-galactopyranosyl-(1→4)-β-D- glucopyranoside 3'-sulfate] show a binding specificity to L-selectin ["BIOCHEM. BIOPHY. RES. COM.", Vol. 190, page 426 (1993)]. These results suggest that the selectin adhesion inhibitor may be employed as an effective ingredient of a novel curing or preventive drug for clinical use.

An object of the present invention is to provide a novel selectin adhesion inhibiting compound which shows excellent inflammatory effect, has excellent safety in use and stability, and can be produced in commercial scale to use the same as an effective ingredient of a preventing or curing drug for inflammatory diseases on vascular wall as well as curing drugs on ARDS, septicemia, rheumatism, shock, nephritis and the like.

As a solution, in accordance with the present invention novel sulfated and phosphated saccharide derivatives are provided which are suitable for attaining the above object.

According to the invention, the object can be attained by sulfated and phosphated saccharide derivatives shown by formula (I):

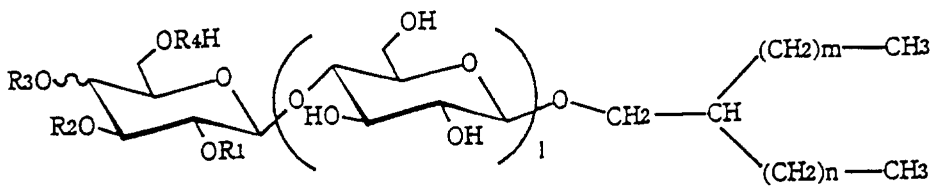

(I)

wherein $R_1$ is hydrogen atom or a residue of sulfate, phosphate or L-fucose; $R_2$, $R_3$ and $R_4$ are hydrogen atom or a residue of sulfate or phosphate, respectively; l is an integer of 0 or 1; m is an integer of 0 - 15; and n is an integer of 0 - 21.
or a pharmaceutically acceptable salt thereof.

As examples of the β-D-galactose derivatives and β-D-lactose derivatives of compound (I), the following compounds are listed: 2-methylpropylβ-D-galactopyranoside 3-sulfate, 2-methylpropylβ-D-galactopyranoside 6-sulfate, 2-methylpropyl β-D-galactopyranoside 3,6-disulfate, 2-methylpropyl β-D-galactopyranoside 3,4,6-trisulfate, 2-methylpropyl β -D-galactopyranoside 2,3,4,6-tetrasulfate, 2-propylpentyl β-D-galactopylanoside 3-sulfate, 2-propylpentyl β -D-galactopyranoside 6-sulfate, 2-propylpentyl β -D-galactopyranoside 3,6-disulfate, 2-propylpentyl β -D-galactopyranoside 2,3,6-trisulfate, 2-propylpentyl β -D-galactopyranoside 2,3,4,6-tetrasulfate, 2-hexadesyltetracosyl β -D-galactopyranoside 3-sulfate, 2-methylpropyl 0-β -D-galactopyranosyl-(1→4)-β-D-glucopyranoside 3'-sulfate, 2-methylpropyl 0-β-D-galactopyranosyl-(1→4)-β-D-glucopyranoside 3',6'-disulfate, 2-propylpentyl 0-β-D-galactopyranosyl-(1→4)-β -D-glucopyranoside 3'-sulfate, 2-propylpentyl 0-β -D-galactopyranosyl-(1→4)-β-D-glucopyranoside 3',6'-disulfate, 2-tetradesylhexadesyl 0-β-D-galactopyranosyl-(1→4)-β -D-glucopyranoside 3',6'-disulfate, 2-tetradesylhexadesyl 0-β -D-galactopyranosyl-(1→4)-β-D-glucopyranoside 3',6',6-trisulfate, 2-methylpropyl β-D-galactopyranoside 2,3,4,6-tetraphosphate, 2-propylpentyl β-D-galactopyranoside 2,3,4,6-tetraphosphate, 2-tetradesylhexadesyl β-D-galactopyranoside 2,3,4,6-tetraphosphate, 2-methylpropyl β-D-galactopyranoside 3,4-diphosphate, 2-propylpentyl β-D-galactopyranoside 3,4-diphosphate, 2-tetradesylhexadesyl β-D-galactopyranoside 3,4-diphosphate, 2-propylpentyl β-D-glucopyranoside 3-triphosphate, 2-propylpentyl β-D-glucopyranoside 2,3-diphosphate, 2-propylpentyl β-D-galactopyranoside 2,3,4-triphosphate, 2-propylpentyl 0-β-D-galactopyranosyl-(1→4)-β-D-glucopyranoside 3',4'-diphosphate, 2-tetradesylhexadesyl 0-β -D-galacto-pyranosyl-(1→4)-β-D-glucopyranoside 3',4'-diphosphate, 2-propylpentyl 0-α-L-fucopyranosyl-(1→2)-β-D-galactopyranoside 3-sulfate, 2-propylpentyl 0-α-L-fucopyranosyl-(1→2)-β -D-galactopyranoside 3,3'-disulfate, 2-propylpentyl 0-α -L-fucopyranosyl-(1→2)-β-D-galactopyranoside 3,6-disulfate, 2-propylpentyl 0-α-L-fucopyranosyl-(1→2)-β-D-galactopyranoside 3,3',6-trisulfate, 2-tetradesylhexadesyl 0-α-L-fucopyranosyl-(1→ 2)-β-D-galactopyranoside 3-sulfate, 2-tetradesylhexadesyl 0-α -L-fucopyranosyl-(1→2)-β-D-galactopyranoside 3,3'-disulfate, 2-tetradesylhexadesyl 0-α-L-fucopyranosyl-(1→2)-β -D-galactopyranoside 3,6-disulfate, 2-tetradesylhexadesyl 0-α -L-fucopyranosyl-(1→2)-β-D-galactopyranoside 3,3',6-trisulfate, 2-propylpentyl 0-α-L-fucopyranosyl-(1→2)-β-D-galactopyranoside 3-phosphate, 2-propylpentyl 0-α-L-fucopyranosyl-(1→2)-β -D-galactopyranoside 3,3'-diphosphate, 2-tetradesylhexadesyl 0-α -L-fucopyranosyl-(1→2)-β-D-galactopyranoside 3-phosphate, 2-tetradesylhexadesyl 0-α-L-fucopyranosyl-(1→2)-β -D-galactopyranoside 3,3'-diphosphate and salts thereof.

According to a process of the invention, the derivatives shown by Formula (I) and salts thereof can be prepared by reacting a compound shown by Formula (II)

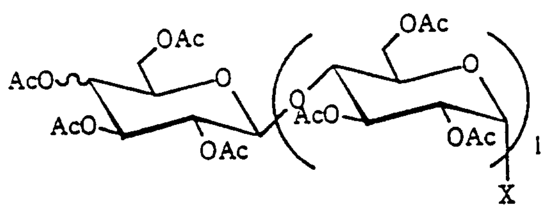

(II)

wherein l has the meaning as referred to; and X is a removable group,
and a compound shown by Formula (III)

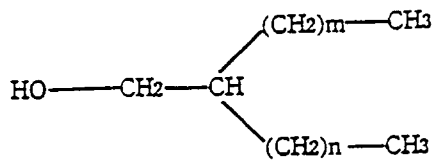

(III)

wherein m and n have the meanings as referred to, reacting the resulting saccharide derivative shown by Formula (IV)

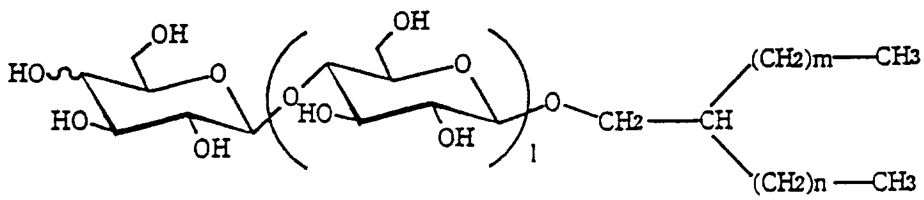

(IV)

wherein l, m and n have the meanings as referred to, with a complex of sulfite and trimethyl ammonium or with the complex after having reacted with di-n-butyl-tin oxide to make the saccharide derivative into its tin-acetal derivative, in case of inserting a sulfate group in desired position; or reacting the saccharide derivative, if necessary, with 2,2-dimethoxy propane and then with a benzyl halide to protect a group in desired position in a conventional manner, removing the protection group, and then reacting with dibenzoyloxy (diisopropylamino) phosphine and 1H-tetrazole, in case of inserting a phosphate group in desired position, and if necessary, converting the resulting sulfated or phosphated saccharide derivative into the salt.

The salt of the compound shown by Formula (I) means, of course, pharmaceutically acceptable one, and sodium, potassium and the like alkali metals as well as ammonium and the like can be listed as actions for forming the salt.

The reaction of the compound (II) (a residue of glycosylhalide with bromine, chlorine, fluorine or the like and glycosylimidate with trichloroacetoimino, N-methylacetoimino or the like radical can be listed as the removable group) with the compound (III) can be carried out by stirring for 0.5 - 24 hours at -30 - +150°C in an inert solvent. Such a solvent may be exemplary listed as benzene, toluene, xylene or the like aromatic hydrocarbon; diethylether, tetrahydrofuran, dioxane or the like ether; methylene chloride, chloroform or the like halogenated hydrocarbon; ethyl acetate, acetonitrile, dimethylformamide, dimethylsulfoxide, acetone or the like as well as a mixture of these solvents. For this reaction, quarternary ammonium salt; mercury bromide, silver trifluoromethanesulfonate, mercury perchloric silver or the like heavy metal salt; boron trifluoride ethyl etherate, trimethylsilyltriflate or the like can be used as an activating agent. Subsequent reaction for removing the protection group is carried out in the presence of a base such as sodium carbonate, potassium carbonate or the like alkaline carbonate; sodium bicarbonate or the like alkaline bicarbonate; sodium hydroxide, potassium hydroxide or the like alkaline hydroxide; triethylamine or the like t-amine.

In case of introducing a residue of sulfate, the resulting saccharide derivative shown by Formula (IV) {however, the compound, wherein l, m, and n are 0, respectively has been known ["CARBOHYD. RES. "Vol. 25, page 59 (1972)]} is reacted with di-n-butyl-tin oxide in an inert solvent such as benzene, toluene, xylene or the like aromatic hydrocarbon; diethylether, tetrahydrofuran, dioxane or the like ether; methylene chloride, chloroform or the like halogenated hydrocarbon; methanol, ethanol, or the like alcohol; ethyl acetate, acetonitrile, dimethylformamide, dimethylsulfoxide, acetone or the like and using a moisture removing fractional distillation equipment to make the saccharide derivative into its tin-acetal derivative, and further reacting with a suitable amount of a complex of sulfite and trimethyl ammonium.

While, in case of introducing a residue of phosphate, the saccharide derivative shown by Formula (IV) is acetonated by using 2,2-dimethoxy propane to protect remaining hydroxy radicals with a residue of benzyl bromide, if necessary, in an inert solvent such as benzene, toluene xylene or the like aromatic hydrocarbon; diethylether, tetrahydrofuran, dioxane or the like ether; methylene chloride, chloroform or the like halogenated hydrocarbon; ethyl acetate, acetonitrile, dimethylformamide, dimethylsulfoxide, acetone or the like, hydrolyzing isopropyridene radical with trifluoroacetic acid, and then reacting with the resulting protected compound with dibenzyloxy (diisopropylamino) phosphine and 1H-tetrazole.

When a medicine shall be prepared by using the compound (I) or salt thereof as an effective ingredient, there is no limitation in form of the medicine and thus it can be made into a tablet, pill, capsule, powder, granule, suppository or the like solid preparation; or a solution, suspension, emulsion or the like liquid preparation. For preparing the solid preparation, starch, lactose, glucose, calcium phosphate, magnesium stearate, carboxymethyl cellulose, or the like filler can

be used and if necessary, a lubricant, disintegrator, coating agent, coloring matter and the like may also be used. The liquid preparation may contain a stabilizer, dissolution aid, suspending agent, emulsifier, buffer, preservative and the like. In view of stability, the liquid preparation may be charged into a vial or the like and then lyophilized for preservation. The lyophilized powder is dissolved into refined water, when it shall be used. Furthermore, the liquid preparation nay contain an isotonic agent, stabilizer, preservative, analgesic additive or the like.

The amount of the compound (I) or salt thereof in the dose depends on the kind of the compound, form of the medicine, extent of disease, age of a patient and other factors, but in general, a range of about 0.1 - 100mg/day is preferable for an adult.

The invention will now be further explained in more detail with reference to Manufacturing Examples, Pharmacological Test Example and Medicine Preparation Examples.

Example 1

Sodium 2-methylpropyl β-D-galactopyranoside 3-sulfate (1-1) and disodium 2-methylpropyl β-D-galactopyranoside 3,6-disulfate (1-2)

A mixture of 2-methylpropyl β-D-galactopyranoside (100mg), di-n-butyl-tin oxide (116mg) in toluene (10ml) was refluxed for 16 hours while removing moisture by fractional distillation and the solvent was distilled out in vacuo. To the residue, N,N-di-methylformamide (1ml) and a complex of sulfurous acid and trimethyl ammonium (70.8mg) were added to stir for 6 hours at room temperature, methanol (4ml) was added thereto, and then the solvent was distilled out in vacuo. After separation and refining by silica-gel chromatography (methanol : chloroform = 1 : 1), the residue was treated with cation exchange resin [AG 50W-X8 Resin (Bio-Rad Co.) 1 x 5cm], the solvent was distilled out in vacuo, and then lyophilized to afford the desired compounds of sodium 2-methylpropyl β -D-galactopyranoside 3-sulfate (52mg) and disodium 2-methylpropyl β -D-galactopyranoside 3,6-disulfate (15mg).

Compound 1 - 1

Mass spectrum ($FAB^-$) m/z :

315 $(M-Na)^-$, 337 $(M-H)^-$.

$^1$H-NMR (270MHz) spectrum ($CD_3OD$) δ ppm :

0.92 (3H, d, J=6.8Hz, $CH_3$),
0.93 (3H, d, J=6.8Hz, $CH_3$),
1.8 - 2.0 (1H, m, $Me_2C\underline{H}$),
3.31 (1H, dd, J=7.8. 9.8Hz, $Me_2CHC\underline{H}aHb$),
3.53 (1H, dt, J=1.0, 6.3Hz, H5),
3.66 (1H, dd, J=7.8, 9.8Hz, $Me_2CHCHa\underline{H}b$),
3.69 (1H, dd, J=7.8, 8.8Hz, H2),
3.73 (2H, d, J=6.3Hz, H6, H6'),
4.21 (1H, dd, J=3.4, 8.8Hz, H3),
4.24 (1H, dd, J=1.0, 3.4Hz, H4),
4.29 (1H, d, J=7.8Hz, H1).

Compound 1 - 2

Mass spectrum ($FAB^-$) m/z :

417 $(M-Na)^-$, 439 $(M-H)^-$ .

$^1$H-NMR (270MHz) spectrum ($CD_3OD$) δ ppm :

0.91 (3H, d, J=6.4Hz, $CH_3$),
0.92 (3H, d, J=6.4Hz, $CH_3$),
1.8 - 2.0 (1H, m, $Me_2C\underline{H}$),
3.31 (1H, dd, J=6.8, 9.3Hz, $Me_2CHC\underline{H}aHb$),
3.65 (1H, dd, J=6.8, 9.3Hz, $Me_2CHCHa\underline{H}b$),
3.69 (1H, dd, J=7.8, 9.3Hz, H2),
3.81 (1H, t, J=6.4Hz, H5),
4.14 (1H, dd, J=6.4. 10.3Hz, H6'),
4.20 (1H, dd, J=6.4, 10.3Hz, H6),
4.23 (1H, d, J=9.3Hz, H3),
4.26 (1H, br, H4),
4.31 (1H, d, J=7.8Hz, H1).

Example 2

Sodium 2-methylpropyl β-D-galactopyranoside 6-sulfate

To 2-methylpropyl β-D-galactopyranoside (100mg) in N,N-dimethylformamide solution (1ml), a complex of sulfurous acid and trimethyl ammonium (161mg) was added to stir for 21 hours at room temperature, and the solvent was distilled out in vacuo. After separation and purification by silica gel chromatography (methanol: chloroform : water = 5 : 8 : 1), the residue was treated with cation exchange resin [AG 50W-X8 Resin (Bio-Rad Co.) 1 x 5cm], the solvent was distilled out in vacuo, and then lyophilized to afford the title compound (76mg).

Mass spectrum ($FAB^-$) m/z :

315 $(M-Na)^-$, 337 $(M-H)^-$.

$^1$H-NMR (270MHz) spectrum ($CD_3OD$) δ ppm :

0.92 (3H, d, J=6.8Hz, $CH_3$),
0.93 (3H, d, J=6.8Hz, $CH_3$),
1.8 - 2.0 (1H, m, $Me_2C\underline{H}$),
3.28 (1H, dd, J=6.8, 9.8Hz, $Me_2CHC\underline{H}aHb$),
3.46 (1H, dd, J=2.9, 9.8Hz, H3),
3.51 (1H, dd, J=6.3, 9.8Hz, H2),
3.65 (1H, dd, J=2.9, 9.8Hz, $Me_2CHCHa\underline{H}b$),
3.75 (1H, dt, J=1.0, 6.4Hz, H5),
3.87 (1H, dd, J=1.0, 2.9Hz, H4),
4.11 (1H, dd, J=6.4, 10.3Hz, H6'),
4.18 (1H, dd, J=6.4, 10.3Hz, H6),
4.20 (1H, d, J=6.3Hz, H1).

Example 3

Trisodium 2-methylpropyl β-D-galactopyranoside3,4,6-trisulfate (3-1) and tetrasodium 2-methylpropyl β-D-galactopyranoside 2,3,4,6-tetrasulfate (3-2)

By treating as described in Example 2 except that 2-methyl propyl β-D-galactopyranoside (100mg) and a complex of sulfurous acid and trimethyl ammonium (500mg) were selected, the title compound of trisodium 2-methylpropyl β-D-galactopyranoside 3,4,6-trisulfate (24mg) and tetrasodium 2-methylpropyl β-D-galactopyranoside 2,3,4,6-tetrasulfate (33mg) were obtained.

Compound 3 - 1

Mass spectrum ($FAB^-$) m/z :

519 $(M-Na)^-$, 541 $(M-H)^-$.

$^1$H-NMR (270MHz) spectrum ($CD_3OD$) δ ppm :

0.92 (3H, d, J=6.8Hz, $CH_3$),
0.93 (3H, d, J=6.8Hz, $CH_3$),
1.8 - 2.0 (1H, m, $Me_2C\underline{H}$),
3.32 (1H, dd, J=6.8, 9.8Hz, $Me_2CHC\underline{H}aHb$),
3.65 (1H, dd, J=7.8, 9.8Hz, H2),
3.66 (1H, dd, J=7.3, 9.8Hz, $Me_2CHCHa\underline{H}b$),
3.95 (1H, dd, J=3.9, 7.8Hz, H5),
4.20 (1H, dd, J=7.8, 11.7Hz, H6'),
4.32 (1H, dd, J=3.4, 9.8Hz, H3),
4.34 (1H, d, J=7.8Hz, H1),
4.40 (1H, dd, J=3.9, 11.7Hz, H6),
4.99 (1H, d, J=3.4Hz, H4).

Compound 3 - 2

Mass spectrum ($FAB^-$) m/z :

621 $(M-Na)^-$.

$^1$H-NMR (270MHz) spectrum ($CD_3OD$) δ ppm :

0.92 (3H, d, J=6.8Hz, $CH_3$),
0.94 (3H, d, J=6.8Hz, $CH_3$),
1.8 - 2.0 (1H, m, $Me_2C\underline{H}$),
3.32 (1H, dd, J=6.9, 9.3Hz, $Me_2CHC\underline{H}aHb$),
3.67 (1H, dd, J=6.4, 9.3Hz, $Me_2CHCHa\underline{H}b$),
4.01 (1H, dd, J=3.9, 7.3Hz, H5),
4.21 (1H, dd, J=7.3, 11.7Hz, H6'),
4.38 (1H, dd, J=3.9, 11.7Hz, H6),
4.44 (1H, d, J=8.3Hz, H3),
4.49 (1H, d, J=3.9Hz, H1),
4.51 (1H, dd, J=3.9, 8.3Hz, H2),
5.09 (1H, br, H4).

Example 4

Sodium 2-propylpentyl β-D-galactopyranoside 3-sulfate

By treating as described in Example 1 except that 2-propylpentyl β-D-galactopyranoside (92mg) and a complex of sulfurous acid and trimethyl ammonium (52.6mg) were selected, the titled compound (71mg) was obtained.

Mass spectrum ($FAB^-$) m/z :
371 $(M-Na)^-$, 393 $(M-H)^-$.

$^1H$-NMR (270MHz) spectrum ($CD_3OD$) δ ppm :
0.89 (6H, t, J=6.8Hz, $CH_3$),
1.2 - 1.4 (8H, m, $C\underline{H}_2C\underline{H}_2$),
1.6 - 1.7 (1H, m, $Pr_2C\underline{H}$),
3.41 (1H, dd, J=5.9, 9.3Hz, $Pr_2CHC\underline{H}aHb$),
3.52 (1H, dt, J=1.0, 6.3Hz, H5),
3.69 (1H, dd, J=7.8, 9.3Hz, H2),
3.73 (2H, d, J=6.3Hz, H6, H6'),
3.81 (1H, dd, J=6.4, 9.3Hz, $Pr_2CHCHa\underline{H}b$),
4.21 (1H, dd, J=3.4, 9.3Hz, H3),
4.24 (1H, dd, J=1.0, 3.4Hz, H4),
4.27 (1H, d, J=7.8Hz, H1).

$^{13}C$-NMR (68MHz) spectrum ($CD_3OD$) δ ppm :
14.8 ($CH_3$),
20.9 ($CH_2\underline{C}H_2CH_3$),
34.7 ($\underline{C}H_2CH_2CH_3$),
39.2 ($Pr_2\underline{C}H$),
62.3 (C6),
68.5 (C4),
70.2 (C2),
73.9 ($Pr_2CH\underline{C}H_2$),
76.2 (C5),
82.4 (C3),
105.1 (C1).

Example 5

Sodium 2-propylpentyl β-D-galactopyranoside 6-sulfate

By treating as described in Example 2 except that 2-propylpentyl β-D-galactopyranoside (100mg) and a complex of sulfurous acid and trimethyl ammonium (56mg) were selected, the title compound (59mg) was obtained.

Mass spectrum ($FAB^-$) m/z :
371 $(M-Na)^-$, 393 $(M-H)^-$.

$^1H$-NMR (270MHz) spectrum ($CD_3OD$) δ ppm :
0.89 (6H, t, J=6.8Hz, $CH_3$),
1.2 - 1.4 (8H, m, $C\underline{H}_2C\underline{H}_2$),

1.6 - 1.7 (1H, m, $Pr_2C\underline{H}$),
3.38 (1H, dd, J=5.9, 9.8Hz, $Pr_2CHC\underline{H}aHb$),
3.45 (1H, dd, J=2.0, 9.8Hz, H3),
3.50 (1H, dd, J=7.3, 9.8Hz, H2),
3.75 (1H, t, J=6.4Hz, H5),
3.78 (1H, dd, J=6.3, 9.8Hz, $Pr_2CHCHa\underline{H}b$),
3.87 (1H, d, J=2.0Hz, H4),
4.10 (1H, dd, J=6.4, 10.3Hz, H6'),
4.18 (1H, dd, J=6.4, 10.3Hz, H6),
4.18 (1H, d, J=7.3Hz, H1).

$^{13}C$-NMR (68MHz) spectrum ($CD_3OD$) δ ppm :

14.8 ($CH_3$),
20.9 ($CH_2\underline{C}H_2CH_3$),
34.6 ($C\underline{H_2}CH_2CH_3$),
39.2 ($Pr_2\underline{C}H$),
67.4 (C6),
69.9 (C4),
72.5 (C2),
73.8 ($Pr_2CH\underline{C}H_2$),
74.1 (C5),
74.8 (C3),
105.3 (C1).

Example 6

Disodium 2-propylpentyl β-D-galactopyranoside 3,6-disulfate

By treating as described in Example 2 except that 2-propylpentyl β-D-galactopyranoside (104mg) and a complex of sulfurous acid and trimethyl ammonium (161mg) were selected, the title compound (70mg) was obtained.

Mass spectrum ($FAB^-$) m/z :

473 $(M-Na)^-$, 495 $(M-H)^-$.

$^1H$-NMR (270MHz) spectrum ($CD_3OD$) δ ppm :

0.89 (6H, t, J=6.8Hz, $CH_3$),
1.2 - 1.4 (8H, m, $C\underline{H_2}C\underline{H_2}$),
1.6 - 1.7 (1H, m, $Pr_2C\underline{H}$),
3.41 (1H, dd, J=5.4, 9.3Hz, $Pr_2CHC\underline{H}aHb$),
3.68 (1H, dd, J=7.8, 8.8Hz, H2),
3.78 (1H, t, J=6.4Hz, H5),
3.79 (1H, dd, J=5.9, 9.3Hz, $Pr_2CHCHa\underline{H}b$),
4.13 (1H, dd, J=6.4, 10.3Hz, H6'),
4.21 (1H, dd, J=6.4, 10.3Hz, H6),
4.21 (1H, dd, J=1.0, 8.8Hz, H3),
4.26 (1H, d, J=1.0Hz, H4),
4.29 (1H, d, J=7.8Hz, H1).

$^{13}C$-NMR (68MHz) spectrum ($CD_3OD$) δ ppm :

14.8 ($CH_3$),
21.0 ($CH_2\underline{C}H_2CH_3$),
34.7 ($\underline{C}H_2CH_2CH_3$),
39.2 ($Pr_2\underline{C}H$),
67.6 (C6),
68.5 (C4),
70.7 (C2),
73.9 ($Pr_2CH\underline{C}H_2$),
73.9 (C5),
81.8 (C3),
105.0 (C1).

Example 7

Trisodium 2-propylpentyl β-D-galactopyranoside 2,3,6-trisulfate (7-1) and tetra 2-propylpentyl β-D-galactopyranoside 2,3,4,6-tetra-sulfate (7-2)

By treating as described in Example 2 except that 2-propylpentyl β-D-galactopyranoside (102mg) and a complex of sulfurous acid and trimethyl ammonium (251mg) were selected, the title compound of trisodium 2-propylpentyl β-D-galactopyranoside 2,3,6-trisulfate (50mg) and tetrasodium 2-propylpentylβ -D-galactopyranoside 2,3,4,6-tetrasulfate (128mg) were obtained.

Compound 7 - 1

Mass spectrum ($FAB^-$) m/z :
575 $(M-Na)^-$, 597 $(M-H)^-$.

$^1$H-NMR (270MHz) spectrum ($CD_3OD$) δ ppm :
0.89 (6H, t, J=6.8Hz, $CH_3$),
1.2 - 1.5 (8H, m, $C\underline{H}2C\underline{H}_2$),
1.6 - 1.7 (1H, m, $Pr_2C\underline{H}$),
3.44 (1H, dd, J=5.9, 9.8Hz, $Pr_2CHC\underline{H}aHb$),
3.77 (1H, dd, J=5.9, 9.8Hz, $Pr_2CHCHa\underline{H}b$),
3.82 (1H, t, J=6.4Hz, H5),
4.14 (1H, dd, J=6.4, 10.7Hz, H6'),
4.21 (1H, dd, J=6.4, 10.7Hz, H6),
4.37 (1H, d, J=8.3Hz, H3),
4.38 (1H, br, H4),
4.43 (1H, d, J=7.3Hz, H1),
4.47 (1H, dd, J=7.3, 8.3Hz, H2).

$^{13}$C-NMR (68MHz) spectrum ($CD_3OD$) δ ppm :
14.8 ($CH_3$),
20.9 ($CR_2\underline{C}H_2CH_3$),
34.4 ($\underline{C}H_2CH_2CH_3$),
39.0 ($Pr_2\underline{C}H$),
67.4 (C6),
68.5 (C4),
73.7 (C5),
73.9 ($Pr_2CH\underline{C}H_2$),
77.1 (C2),
80.0 (C3),
103.4 (C1).

Compound 7 - 2

Mass spectrum ($FAB^-$) m/z :
677 $(M-Na)^-$.

$^1$H-NMR (270MHz) spectrum ($CD_3OD$) δ ppm :
0.89 (6H, t, J=6.4Hz, $CH_3$),
1.2 - 1.5 (8H, m, $C\underline{H}_2C\underline{H}_2$),
1.6 - 1.7 (1H, m, $Pr_2C\underline{H}$),
3.43 (1H, dd, J=6.4, 9.3Hz, $Pr_2CHC\underline{H}aHb$),
3.79 (1H, dd, J=5.9, 9.3Hz, $Pr_2CHCHa\underline{H}b$),
4.00 (1H, dd, J=4.4, 7.3Hz, H5),
4.20 (1H, dd, J=7.3, 11.2Hz, H6'),
4.38 (1H, dd, J=4.4, 11.2Hz, H6),
4.4 - 4.5 (1H, m, H2),
4.4 - 4.5 (1H, m, H3),
4.47 (1H, br, H1),
5.10 (1H, br, H4).

$^{13}$C-NMR (68MHz) spectrum ($CD_3OD$) δ ppm :
14.8 ($CH_3$),

20.9 ($CH_2\underline{C}H_2CH_3$),
34.4 ($\underline{C}H_2CH_2CH_3$),
39.0 ($Pr_2\underline{C}H$),
68.4 (C6),
73.5 (C5),
73.8 ($Pr_2CH\underline{C}H_2$),
76.1 (C4),
76.6 (C2),
77.6 (C3),
103.0 (C1).

Example 8

Sodium 2-hexadesyltetracosyl β-D-galactopyranoside 3-sulfate

By treating as described in Example 1 except that 2-hexadesyltetracosyl β-D-galactopyranoside (100mg) and a complex of sulfurous acid and trimethyl ammonium (22.5mg) were selected, the title compound (65mg) was obtained.

Mass spectrum ($FAB^-$) m/z :
820.3 $(M-Na)^-$, 842.3 $(M-H)^-$.

$^1H$-NMR (270MHz) spectrum ($CD_3OD$) δ ppm :
0.88 (6H, t, J=6.8Hz, $CH_3$),
1.27 (72H, s, $C\underline{H_2}$),
1.5 - 1.7 (1H, m, $(C_{16}H_{32})(C_{22}H_{45})C\underline{H}$),
3.42 (1H, dd, J=5.9, 8.9Hz, $(C_{16}H_{32})(C_{22}H_{45})CHC\underline{H}aHb$),
3.63 (1H, t, J=5.6Hz, H5),
3.75 (1H, dd, J=7.9, 12.3Hz, H2),
3.7 - 3.9 (2H, m, H6,H6'),
3.76 (1H, dd, J=5.9, 8.9Hz, $(C_{16}H_{32})(C_{22}H_{45})CHCHa\underline{H}b$),
4.34 (1H, d, J=7.9Hz, H1),
4.42 (1H, dd, J=3.7, 12.3Hz, H3),
4.47 (1H, d, J=3.7Hz, H4).

$^{13}C$-NMR (68MHz) spectrum ($CD_3OD$ - $H_2O$) δ ppm :
13.9 ($CH_3$),
22.7, 26.8, 26.9, 29.4, 29.7,
29.8 29.9, 30.3, 31.2, 32.0 ($CH_2$),
38.5 (CH),
61.1 (C6),
67.1 (C4),
69.5 (C2),
73.6 (C5),
73.6 ($OCH_2$),
81.2 (C3),
103.7 (C1).

Example 9

Sodium 2-methylpropyl 0-β-D-galactopyranosyl-(1→4)-β -D-glucopyranoside 3'-sulfate (9-1) and disodium 2-methylpropyl 0-β-D-galactopyranosyl-(1→4)-β-D-glucopyranoside 3',6'-disulfate (9-2)

By treating as described in Example 1 except that 2-methylpropyl 0-β-D-galactopyranosyl-(1→4)-β-D-glucopyranoside (100mg) and a complex of sulfurous acid and trimethyl ammonium (41.7mg) were selected, the title compound of sodium 2-methylpropyl 0-β-D-galactopyranosyl-(1→4)-β-D-glucopyranoside 3'-sulfate (96mg) and disodium 2-methylpropyl 0-β-D-galactopyranosyl-(1→4)-β -D-glucopyranoside3',6'-disulfate (8mg) were obtained.

Compound 9 - 1

Mass spectrum ($FAB^-$) m/z :
477 $(M-Na)^-$, 499 $(M-H)^-$.

| | |
|---|---|
| $^{1}$H-NMR (270MHz) spectrum (DMSO-$d_6$) δ ppm : | |
| glucose | H1 (4.28, 1H, d, J=7.8Hz), |
| | H2 (3.2 - 3.3, 1H, m), |
| | H3 (3.5 - 3.6, 1H, m), |
| | H4 (3.6 - 3.7, 1H, m), |
| | H5 (3.3 - 3.4, 1H, m), |
| | H6, H6' (3.8 - 3.9, 2H, m), |
| galactose | H1 (4.48, 1H, d, J=7.4Hz), |
| | H2 (3.6 - 3.7, 1H, m), |
| | H3 (4.2 - 4.3, 1H, m), |
| | H4 (4.2 - 4.3, 1H, m), |
| | H5 (3.5 - 3.6, 1H, m), |
| | H6, H6' (3.7 - 3.8, 2H, m), |
| aglycon | $CH_3$ (0.93, 6H, t, J=6.9Hz), |
| | CH (1.9 - 2.0, 1H,m), |
| | OC<u>H</u>aHb (3.3 - 3.4, 1H, m), |
| | OCHa<u>H</u>b (3.6 - 3.7, 1H, m). |
| $^{13}$C-NMR (68MHz) spectrum (DMSO-$d_6$) δ ppm : | |
| glucose | C1 (104.5), |
| | C2 (74.8), |
| | C3 (76.4), |
| | C4 (81.2), |
| | C5 (76.4), |
| | C6 (62.0). |
| galactose | C1 (105.0), |
| | C2 (70.9), |
| | C3 (81.8), |
| | C4 (68.6), |
| | C5 (76.8), |
| | C6 (62.4), |
| aglycon | $CH_3$ (19.6), |
| | CH (29.7), |
| | $OCH_2$ (77.6). |

<u>Compound 9 - 2</u>

| | |
|---|---|
| Mass spectrum (FAB $^-$) m/z : | |
| | 579 $(M-Na)^-$. |
| $^{1}$H-NMR (270MHz) spectrum (DMSO-$d_6$) δ ppm : | |
| glucose | H1 (4.29, 1H, d, J=7.9Hz), |
| | H2 (3.2 - 3.3, 1H, m), |
| | H3 (3.5 - 3.6, 1H, m), |
| | H4 (3.6 - 3.7, 1H, m), |
| | H5 (3.3 - 3.4, 1H, m), |
| | H6, H6' (3.8 - 3.9, 2H, m), |
| galactose | H1 (4.50, 1H, d, J=7.6Hz), |
| | H2 (3.6 - 3.7, 1H, m), |
| | H3 (4.2 - 4.3, 1H, m), |
| | H4 (4.2 - 4.3, 1H, m), |
| | H5 (3.7 - 3.8, 1H, m), |
| | H6, H6' (4.0 - 4.2, 2H, m), |
| aglycon | $CH_3$ (0.93, 6H, t, J=6.9Hz), |
| | CH (1.9 - 2.0, 1H,m), |
| | OC<u>H</u>aHb (3.3 - 3.4, 1H, m), |
| | OCHa<u>H</u>b (3.6 - 3.7, 1H, m). |
| $^{13}$C-NMR (68MHz) spectrum (DMSO-$d_6$) δ ppm : | |
| glucose | C1 (104.7), |
| | C2 (75.0), |
| | C3 (76.5), |

| | |
|---|---|
| | C4 (81.1), |
| | C5 (76.4), |
| | C6 (62.2), |
| galactose | C1 (104.9), |
| | C2 (70.8), |
| | C3 (81.8), |
| | C4 (68.6), |
| | C5 (74.8), |
| | C6 (66.4), |
| aglycon | $CH_3$ (19.6), |
| | CH (29.7), |
| | $OCH_2$ (77.7). |

Example 10

Sodium 2-propylpentyl 0-β-D-galactopyranosyl-(1→4)-β-D-glucopyranoside 3'-sulfate (10-1) and disodium 2-propylpentyl 0-β-D-galactopyranosyl-(1→4)-β-D-glucopyranoside3',6'-disulfate (10-2)

By treating as described in Example 1 except that 2-propylpentyl 0-β-D-galactopyranosyl-(1→4)-β-D-glucopyranoside (105mg) and a complex of sulfurous acid and trimethyl ammonium (38.4mg) were selected, the title compound of sodium 2-propylpentyl 0-β-D-galactopyranosyl-(1→4)-β-D-glucopyranoside 3'-sulfate (95mg) and disodium 2-propylpentyl 0-β-D-galactopyranosyl-(1→4)-β -D-glucopyranoside 3',6'-disulfate (7mg) were obtained.

Compound 10 - 1

Mass spectrum ($FAB^-$) m/z :

| | |
|---|---|
| | 533 $(M-Na)^-$, 555 $(M-H)^-$. |
| $^1H$-NMR (270MHz) spectrum (DMSO-$d_6$) δ ppm : | |
| glucose | H1 (4.23, 1H, d, J=7.9Hz), |
| | H2 (3.17, 1H, t, J=7.9Hz), |
| | H3 (3.4 - 3.5, 1H, m), |
| | H4 (3.4 - 3.5, 1H, m), |
| | H5 (3.3 - 3.4, 1H, m), |
| | H6' (3.79, 1H, dd, J=3.0, 12.4Hz), |
| | H6 (3.86, 1H, dd, J=3.0, 12.4Hz), |
| galactose | H1 (4.34, 1H, d, J=7.4Hz), H2 (3.6 - 3.7, 1H, m), |
| | H3 (4.18, 1H, dd, J=3.5, 9.9Hz), |
| | H4 (4.14, 1H, d, J=3.5Hz), |
| | H5 (3.5 - 3.6, 1H, m), |
| | H6, H6' (3.6 - 3.7, 2H, m), |
| aglycon | $CH_3$ (0.89, 6H, t, J=6.9Hz), |
| | $CH_2$ (1.2 - 1.4, 8H, m), |
| | CH (1.5 - 1.7, 1H, m), |
| | OC<u>H</u>aHb (3.37, 1H, dd, J=5.9, 9.9Hz), |
| | OCHa<u>H</u>b (3.75, 1H, dd, J=5.9, 9.9Hz). |
| $^{13}C$-NMR (68MHz) spectrum (DMSO-$d_6$) δ ppm : | |
| glucose | C1 (105.6), |
| | C2 (75.8), |
| | C3 (77.5), |
| | C4 (82.8), |
| | C5 (77.4), |
| | C6 (63.1), |
| galactose | C1 (106.1), |
| | C2 (71.8), |
| | C3 (82.2), |
| | C4 (69.3), |
| | C5 (77.8), |
| | C6 (63.1), |

| | |
|---|---|
| aglycon | $CH_3$ (5.6), |
| | $CH_2$ (11.4, 25.1), |
| | CH (29.5), |
| | $OCH_2$ (74.8). |

Compound 10 - 2

| | |
|---|---|
| Mass spectrum (FAB $^-$) m/z : | |
| | 635 $(M-Na)^-$. |
| $^1$H-NMR (270MHz) spectrum (DMSO-$d_6$) δ ppm : | |
| glucose | H1 (4.24, 1H, d, J=7.9Hz), |
| | H2 (3.15, 1H, t, J=7.9Hz), |
| | H3 (3.4 - 3.5, 1H, m), |
| | H4 (3.4 - 3.5, 1H, m), |
| | H5 (3.3 - 3.4, 1H, m), |
| | H6' (3.76, 1H, dd, J=3.0, 12.4Hz), |
| | H6 (3.89, 1H, dd, J=3.0, 12.4Hz), |
| galactose | H1 (4.35, 1H, d, J=7.5Hz), |
| | H2 (3.6 - 3.7, 1H, m), |
| | H3 (4.20, 1H, dd, J=3.7, 9.9Hz), |
| | H4 (4.19, 1H, d, J=3.7Hz), |
| | H5 (3.7 - 3.8, 1H, m), |
| | H6, H6' (3.9 - 4.1, 2H, m), |
| aglycon | $CH_3$ (0.89, 6H, t, J=6.9Hz), |
| | $CH_2$ (1.2 - 1.4, 8H, m), |
| | CH (1.5 - 1.7, 1H, m), |
| | OC<u>H</u>aHb (3.40, 1H, dd, J=5.9, 9.9Hz), |
| | OCHa<u>H</u>b (3.76, 1H, dd, J=5.9, 9.9Hz). |
| $^{13}$C-NMR (68MHz) spectrum (DMSO-$d_6$) δ ppm : | |
| glucose | C1 (105.5), |
| | C2 (75.9), |
| | C3 (77.5), |
| | C4 (82.8), |
| | C5 (77.5), |
| | C6 (63.2), |
| galactose | C1 (106.1), |
| | C2 (71.5), |
| | C3 (82.3), |
| | C4 (69.1), |
| | C5 (75.8), |
| | C6 (67.5), |
| aglycon | $CH_3$ (5.6), |
| | $CH_2$ (11.4, 25.1), |
| | CH (29.6), |
| | $OCH_2$ (74.8). |

Example 11

Disodium 2-tetradecylhexadecyl 0-β-D-galactopyranosyl-(1→ 4)-β-D-glucopyranoside 3',6'-disulfate (11-1), trisodium 2-tetradecylhexadecyl 0-β-D-galactopyranosyl-(1→4)-β -D-glucopyranoside 3',6',6-trisulfate (11-2) and sodium 2-tetradecylhexadecyl 0-β-D-galactopyranosyl-(1→4)-β-D-glucopyranoside 3'-sulfate (Na) (11-3)

By treating as described in Example 1 except that 2-tetradecylhexadecyl 0-β-D-galactopyranosyl-(1→4)-β -D-glucopyranoside (115mg) and a complex of sulfurous acid and trimethyl ammonium (46.2mg) were selected. the title compound of disodium 2-tetradecylhexadecyl 0-β-D-galactopyranosyl-(1→4)-β -D-glucopyranoside 3',6'-disulfate (70mg), trisodium 2-tetradecylhexadecyl 0-β-D-galactopyranosyl-(1→4)-β -D-glucopyranoside 3',6',6-trisulfate (30mg) and sodium 2-tetradecylhexadecyl 0-β-D-galactopyranosyl-(1→4)-β -D-glucopyranoside3'-trisulfate (31mg) were obtained.

Compound 11 - 1

Mass spectrum ($FAB^-$) m/z : 943.5 $(M-Na)^-$.

$^1H$-NMR (270MHz) spectrum (DMSO-$d_6$) $\delta$ ppm :

| | |
|---|---|
| glucose | H1 (4.15, 1H, d, J=7.9Hz), |
| | H2 (3.04, 1H, t, J=7.9Hz), |
| | H3 (3.3 - 3.4, 1H, m), |
| | H4 (3.3 - 3.4, 1H, m), |
| | H5 (3.2 - 3.3, 1H, m), |
| | H6 (3.6 - 3.7, 1H, m), |
| | H6' (3.7 - 3.8, 2H, m), |
| galactose | H1 (4.36, 1H, d, J=7.9Hz), |
| | H2 (3.54, 1H, dd, J=9.9Hz), |
| | H3 (4.0 - 4.1, 1H, m), |
| | H4 (3.94, 1H, d, J=2.9Hz), |
| | H5 (3.7 - 3.8, 1H, m), |
| | H6, H6' (3.8 - 3.9, 2H, m), |
| aglycon | $CH_3$ (0.86, 6H, t, J=6.9Hz), |
| | $CH_2$ (1.1 - 1.4, 52H, m), |
| | CH (1.4 - 1.6, 1H, m), |
| | OC<u>H</u>aHb (3.2 - 3.3, 1H, m), |
| | OCHa<u>H</u>b (3.6 - 3.7, 1H, m). |

$^{13}C$-NMR (68MHz) spectrum (DMSO-$d_6$) $\delta$ ppm :

| | |
|---|---|
| glucose | C1 (103.1), |
| | C2 (73.5), |
| | C3 (75.2), |
| | C4 (80.5), |
| | C5 (75.1), |
| | C6 (60.9). |
| galactose | C1 (103.9), |
| | C2 (68.9), |
| | C3 (79.1), |
| | C4 (66.8), |
| | C5 (73.3), |
| | C6 (65.0), |
| aglycon | $CH_3$ (13.9), |
| | $CH_2$ (22.2, 26.2, 28.8, 29.1, 29.5, 30.5, 31.4), |
| | CH (37.7), |
| | $OCH_2$ (72.2). |

Compound 11 - 2

Mass spectrum ($FAB^-$) m/z : 1045.4 $(M-Na)^-$.

$^1H$-NMR (270MHz) spectrum (DMSO-$d_6$) $\delta$ ppm :

| | |
|---|---|
| glucose | H1 (4.18, 1H, d, J=7.8Hz), |
| | H2 (3.07, 1H, t, J=7.8Hz), |
| | H3 (3.3 - 3.4, 1H, m), |
| | H4 (3.3 - 3.4, 1H, m), |
| | H5 (3.4 - 3.5, 1H, m), |
| | H6 (3.9 - 4.0, 1H, m), |
| | H6' (4.0 - 4.1, 2H, m), |
| galactose | H1 (4.43, 1H, d, J=8.3), |
| | H2 (3.4 - 3.5, 1H, m), |
| | H3 (4.0 - 4.1, 1H, m), |
| | H4 (3.9 - 4.0, 1H, m), |
| | H5 (3.7 - 3.8, 1H, m), |
| | H6, H6' (3.8 - 4.0, 2H, m), |

aglycon $CH_3$ (0.86, 6H, t, J=6.6Hz),
$CH_2$ (1.1 - 1.4, 52H, m),
CH (1.4 - 1.6, 1H, m),
OCHaHb (3.2 - 3.3, 1H, m),
OCHaHb (3.6 - 3.7, 1H, m).

$^{13}$C-NMR (68MHz) spectrum (DMSO-$d_6$) δ ppm :

glucose C1 (103.0), C2 (73.1),
C3 (74.9),
C4 (80.3),
C5 (73.1),
C6 (65.2),

galactose C1 (103.5),
C2 (69.1),
C3 (78.6),
C4 (66.7),
C5 (73.3),
C6 (65.0),

aglycon $CH_3$ (13.9),
$CH_2$ (22.2, 26.2, 28,8, 29.2, 29.5, 30.6, 31.4),
CH (37.8),
$OCH_2$ (72.4).

<u>Compound 11 - 3</u>

Mass spectrum (FAB$^-$) m/z :
841.5 (M-Na)$^-$, 863.5 (M-H)$^-$.

$^1$H-NMR (270MHz) spectrum (DMSO-$d_6$) δ ppm :

glucose H1 (4.13, 1H, d, J=7.4Hz),
H2 (3.00, 1H, t, J=7.4Hz),
H3 (3.1 - 3.4, 1H, m),
H4 (3.2 - 3.4, 1H, m),
H5 (3.1 - 3.4, 1H, m),
H6, H6' (3.5 - 3.8, 2H, m),

galactose H1 (4.33, 1H, d, J=7.4Hz),
H2 (3.4 - 3.6, 1H, m),
H3 (4.01, 1H, dd, J=3.5, 9.9Hz),
H4 (3.89, 1H, d, J=3.5Hz),
H5 (3.4 - 3.6, 1H, m),
H6, H6' (3.3 - 3.7, 2H, m),

aglycon $CH_3$ (0.85, 6H, t, J=6.9Hz),
$CH_2$ (1.30, 52H, s),
CH (1.5 - 1.7, 1H, m),
OCHaHb (3.2 - 3.3, 1H, m),
OCHaHb (3.6 - 3.7, 1H, m).

$^{13}$C-NMR (68MHz) spectrum (DMSO-$d_6$) δ ppm :

glucose C1 (102.9),
C2 (73.1),
C3 (74.9),
C4 (81.1),
C5 (74.7),
C6 (60.5),

galactose C1 (103.7),
C2 (69.3),
C3 (78.7),
C4 (66.5),
C5 (75.3),
C6 (60.0),

aglycon $CH_3$ (13.8),
$CH_2$ (22.0, 25.9, 28,6, 28.9, 29.3, 30.3, 31.2),

CH (37.5),
$OCH_2$ (72.0).

Example 12

Octasodium 2-methylpropyl β-D-galactopyranoside 2,3,4,6-tetraphosphate

To a suspension of 2-methylpropyl β-D-galactopyranoside (100mg) and 1H-tetrazole (356mg) in a mixture of dichloromethane and acetonitrile (1 : 1, 5ml), dibenzyloxy (diisopropylamino) phosphine (1.17g) was added to stir for 2 hours at room temperature under argon atmosphere. Then, water (10ml), ruthenium chloride (2mg) and sodium periodate (724mg) were added to stir for 12 hours. To the reaction mixture, methylene chloride (20ml) was added to obtain an organic layer which was dried over anhydrous sodium sulfate, and the solvent was distilled out in vacuo. The residue was separated and purified by silica-gel chromatography (ethyl acetate : hexane = 1 : 2). To the reaction product in methanol (2ml), water (0.3ml) and 10%-palladium carbon (50mg) were added to stir for 30 minutes at room temperature under hydrogen atmosphere. The pH of the resulting solution was adjusted to 7.0 by adding 0.1N sodium hydroxide solution and further stirred for 24 hours under hydrogen atmosphere. The reaction solution was filtrated with a celite (Johns Manville Sales Corp.), and then the solvent was distilled out in vacuo. The residue was dissolved into water (5ml), treated with cation exchange resin [WK-10 $Na^+$ type, "Diaion" (trademark), 1 x 5cm], and lyophilized to afford the title compound (44mg).

Mass spectrum ($FAB^+$) m/z :
732.9 $(M+H)^+$.

$^1H$-NMR (270MHz) spectrum ($D_2O$) δ ppm :
0.97 (3H, d, J=6.7Hz, $CH_3$),
0.99 (3H, d, J=6.7Hz, $CH_3$),
1.9 - 2.0 (1H, m, $Me_2C\underline{H}$),
3.55 (1H, dd, J=6.7, 9.4Hz, $Me_2CHC\underline{H}aHb$),
3.75 (1H, dd, J=6.7, 9.4Hz, $Me_2CHCHa\underline{H}b$),
3.9 - 4.0 (1H, m, H5),
3.9 - 4.0 (1H, m, H6'),
4.0 - 4.1 (1H, m, H6),
4.2 - 4.3 (1H, m, H3),
4.2 - 4.3 (1H, m, H2),
4.61 (1H, d, J=6.9Hz, H1),
4.63 (1H, dd, J=2.0, 8.9Hz, H4).

$^{13}C$-NMR (68MHz) spectrum ($D_2O$) δ ppm :
20.7 ($CH_3$),
29.8 ($Me_2CH$),
65.8 (C6),
75.0 (C4),
76.3 (C5),
76.7 (C2),
77.6 (C3),
79.3 ($Me_2CH\underline{C}H_2$),
104.4 (C1).

Example 13

Octasodium 2-propylpentyl β-D-galactopyranoside 2,3,4,6-tetraphosphate

By treating as described in Example 12 except that 2-propylpentyl β-D-galactopyranoside (100mg), 1H-tetrazol (285mg) and dibenzyloxy (diisopropylamino) phosphine (939mg) were selected, the title compound (149mg) was obtained.

Mass spectrum ($FAB^+$ ) m/z :
788.9 $(M+H)^+$ .

$^1H$-NMR (270MHz) spectrum ($D_2O$) δ ppm :
0.93 (6H, t, J=6.4Hz, $CH_3$),
1.3 - 1.5 (8H, m, $C\underline{H}_2C\underline{H}_2$),

1.7 - 1.8 (1H, m, $Pr_2C\underline{H}$),
3.64 (1H, dd, J=6.4, 9.4Hz, $Pr_2CHC\underline{H}aHb$),
3.89 (1H, dd, J=6.4, 9.4Hz, $Pr_2CHCHa\underline{H}b$),
3.9 - 4.0 (1H, m, H5),
4.0 - 4.1 (1H, m, H6'),
4.0 - 4.1 (1H, m, H6),
4.2 - 4.3 (1H, m, H3),
4.2 - 4.3 (1H, m, H2),
4.57 (1H, dd, J=2.0, 8.0Hz, H4),
4.58 (1H, d, J=7.4Hz, H1).

$^{13}$C-NMR (68MHz) spectrum ($D_2O$) δ ppm :

15.9 ($CH_3$),
21.2 ($CH_2\underline{C}H_2CH_3$),
34.4 ($C\underline{H_2}CH_2CH_3$),
39.0 ($Pr_2\underline{C}H$),
66.2 (C6),
74.9 (C4),
76.2 ($Pr_2CH\underline{C}H_2$),
76.7 (C2),
76.8 (C5),
77.8 (C3),
104.8 (C1).

Example 14

Octasodium 2-tetradesylhexadesyl β-D-galactopyranoside2,3,4,6-tetraphosphate

By treating as described in Example 12 except that 2-tetradesylhexadesyl β-D-galactopyranoside (100mg), 1H-tetrazol (140mg) and dibenzyloxy (diisopropylamino) phosphine (460mg) were selected, the title compound (69mg) was obtained.

Mass spectrum (FAB $^+$) m/z :

1097.3 $(M+H)^+$.

$^1$H-NMR (270MHz) spectrum ($D_2O$) δ ppm :

0.9 - 1.0 (6H, m, $CH_3$),
1.2 - 1.5 (52H, m, $C\underline{H}2$),
1.7 - 1.8 [(1H, m, $(C_{14}H_{29})_2C\underline{H}$)],
3.5 - 3.6 (1H, m, $(C_{14}H_{29})_2CHC\underline{H}aHb$),
3.8 - 4.0 (1H, m, $(C_{14}H_{29})_2CHCHa\underline{H}b$),
3.9 - 4.0 (1H, m, H5),
3.9 - 4.0 (1H, m, H6'),
4.0 - 4.1 (1H, m, H6),
4.2 - 4.3 (1H, m, H3),
4.2 - 4.3 (1H, m, H2),
4.6 - 4.7 (1H, m, H1),
4.6 - 4.7 (1H, m, H4).

$^{13}$C-NMR (68MHz) spectrum ($D_2O$) δ ppm :

15.9 ($CH_3$),
24 - 32 ($CH_2$),
39.6 [$(C_{14}H_{29})_2\underline{C}H$],
64.6 (C6),
74.6 (C4),
75.4 (C5),
76.7 (C2),
76.9 [$(C_{14}R_{29})_2CH\underline{C}H_2$],
77.6 (C3),
104.7 (C1).

Example 15

Tetrasodium 2-methylpropyl β-D-galactopyranoside 3,4-diphosphate

To 2-methylpropyl β-D-galactopyranoside in acetone (20ml), 97% sulfuric acid (10 μl) was added to stir for 18 hours under argon atmosphere. The reaction solution was neutralized by adding sodium carbonate, filtrated, and the solvent was distilled out in vacuo. The residue was purified by silica-gel chromatography (ethyl acetate : hexane = 3 : 2). To the reaction product in N,N-dimethylformamide (2ml), sodium hydride (73.5mg) was added, and then, benzyl bromide (218 μl) was added thereto under ice cooling to stir for 2 hours at room temperature under argon atmosphere. Under ice cooling, methanol (1ml) and chloroform (10ml) were added to the resulting solution which was washed by water, dried over anhydrous sodium sulfate, and then the solvent was distilled out in vacuo. The reaction product was purified by silica-gel chromatography (ethyl acetate : hexane = 1 : 5). To the purified reaction product in methylene chloride (3ml), 90% trifluoroacetic acid (0.8ml) was added, which was stirred for 1 hour under ice cooling, and then the solvent was distilled out in vacuo. The residue and 1H-tetrazol (82mg) were suspended in a mixture of dichloromethane and acetonitoril (1 : 1, 4ml), and then dibenzyloxy (diisopropylamino) phosphine (539mg) was added to stir for 12 hours at room temperature under argon atmosphere. To the reaction mixture, water (10ml), ruthenium chloride (1mg) and sodium periodate (334mg) were added to stir for 12 hours. By adding methylene chloride (20ml) into the reaction solution, an organic layer was obtained and dried over anhydrous sodium sulfate, and then the solvent was distilled out in vacuo. The residue was purified by silica-gel chromatography (ethyl acetate : hexane = 1 : 1). To the resulting product in methanol (3ml), water (0.5ml) and 10%-palladium carbon (50mg) were added to stir for 30 minutes under hydrogen atmosphere. Resulting solution was neutralized by 0.1N sodium hydroxide solution, stirred for 24 hours at room temperature under hydrogen atmosphere, filtrated with a celite, and then the solvent was distilled out in vacuo. The residue was dissolved into water (5ml) and treated with a cation exchange resin [WK-10 $Na^+$ type "Diaion" (trademark) 1 x 5cm], and lyophilized to afford the title compound (124mg).

Mass spectrum ($FAB^+$) m/z :
485.0 $(M+H)^+$.

$^1$H-NMR (270MHz) spectrum ($D_2O$) δ ppm :
0.96 (3H, d, J=6.9Hz, $CH_3$),
1.9 - 2.0 (1H, m, $Me_2C\underline{H}$),
3.50 (1H, dd, J=6.9, 9.4Hz, $Me_2CHC\underline{H}aHb$),
3.63 (1H, dd, J=6.9, 9.4Hz, $Me_2CHCHa\underline{H}b$),
3.6 - 3.7 (1H, m, H2),
3.6 - 3.7 (1H, m, H6'),
3.7 - 3.8 (1H, m, H6),
3.7 - 3.8 (1H, m, H5),
4.2 - 4.3 (1H, m, H3),
4.54 (1H, d, J=7.9Hz, H1),
4.67 (1H, dd, J=3.0, 9.9Hz, H4).

$^{13}$C-NMR (68MHz) spectrum ($D_2O$) δ ppm :
20.5 ($CH_3$),
29.8 ($Me_2\underline{C}H$),
61.5 (C6),
72.1 (C2),
72.6 (C4),
76.0 (C5),
78.0 (C3),
79.1 ($Me_2CH\underline{C}H_2$),
104.8 (C1).

Example 16

Tetrasodium 2-propylpentyl β-D-galactopyranoside 3,4-diphosphate

By treating as described in Example 15 except that 2-propylpentyl β-D-galactopyranoside (150mg), 1H-tetrazol (74mg) and dibenzyloxy (diisopropylamino) phosphine (485mg) were selected, the title compound (195mg) was obtained.

Mass spectrum ($FAB^+$) m/z :

541.1 $(M+H)^+$.

$^1$H-NMR (270MHz) spectrum ($D_2O$)δ ppm :

0.93 (6H, t, J=6.4Hz, $CH_3$),
1.3 - 1.4 (8H, m, C$\underline{H}_2$C$\underline{H}$2),
1.7 - 1.8 (1H, m, $Pr_2$C$\underline{H}$),
3.62 (1H, dd, J=5.9, 9.9Hz, $Pr_2$CHC$\underline{H}$aHb),
3.74 (1H, dd, J=7.9, 9.4Hz, H2),
3.7 - 3.8 (1H, m, H6'),
3.8 - 3.9 (1H, m, H5),
3.8 - 3.9 (1H, m, H6),
3.8 - 3.9 (1H, dd, J=5.9, 9.9Hz, $Pr_2$CHCHa$\underline{H}$b),
4.2 - 4.3 (1H, m, H3),
4.53 (1H, d, J=7.9Hz, H1),
4.69 (1H, dd, J=2.0, 10.9Hz, H4).

$^{13}$C-NMR (68MHz) spectrum ($D_2O$) δ ppm :

15.7 ($CH_3$),
21.2 ($CH_2\underline{C}H_2CH_3$),
34.6 ($\underline{C}H_2CH_2CH_3$),
38.9 ($Pr_2\underline{C}H$),
61.5 (C6),
72.0 (C2),
72.6 (C4),
75.8 (C5),
75.8 ($Pr_2CH\underline{C}H_2$),
78.1 (C3),
105.0 (C1).

Example 17

Tetrasodium 2-tetradesylhexadesyl β-D-galactopyranoside 3,4-diphosphate

By treating as described in Example 15 except that 2-tetradesylhexadesyl β-D-galactopyranoside (200mg), 1H-tetrazol (36mg) and dibenzyloxy (diisopropylamino) phosphine (236mg) were selected, the title compound (103mg) was obtained.

Mass spectrum ($FAB^+$) m/z :

849.4 $(M+H)^+$.

$^1$H-NMR (270MHz) spectrum ($D_2O$) δ ppm :

0.9 - 1.0 (6H, m, $CH_3$),
1.2 - 1.5 (52H, m, C$\underline{H}_2$),
1.8 - 1.9 (1H, m, $(C_{14}H_{29})_2C\underline{H}$),
3.4 - 3.5 (1H, m, $(C_{14}R_{29})_2$CHC$\underline{H}$aHb),
3.6 - 3.7 (1H, m, H5),
3.7 - 3.8 (1H, m, H2),
3.7 - 3.8 (1H, m, $(C_{14}H_{29})_2$CHCHa$\underline{H}$b),
3.7 - 3.8 (1H, m, H6'),
3.8 - 3.9 (1H, m, H6),
4.2 - 4.3 (1H, m, H3),
4.40 (1H, d, J=7.9Hz, H1),
4.7 - 4.8 (1H, m, H4).

$^{13}$C-NMR (68MHz) spectrum ($D_2O$) δ ppm :

15.8 ($CH_3$),
24 - 35 ($CH_2$),
39.8 $[(C_{14}H_{29})_2C\underline{H}]$,
61.1 (C6),
72.1 (C2),
72.3 (C4),
75.7 $[(C_{14}H_{29})_2CH\underline{C}H_2]$,
75.9 (C5),

78.0 (C3),
105.6 (C1).

Example 18

Disodium 2-propylpentyl β-D-galactopyranoside 3-phosphate

To a mixture of 2-propylpentyl β-D-galactopyranoside (400mg), di-n-butyl-tin oxide (341mg) and benzene (20ml), Molecular Sieves 4A (Linde Co., 400mg,) was added to reflux the same for 18 hours, while removing moisture by a fractional distillation. To the reaction mixture, tetra-n-butylammonium iodide (505mg) and 4-methoxybenzylchloride (463 μl) were added to further reflux for 4 hours and filtered, and then the solvent was distilled out in vacuo. The residue was purified by silica-gel chromatography (ethyl acetate : hexane = 2 : 1). The resulting product was dissolved into N,N-dimethylformamide (3ml), and sodium hydride (175mg) was added thereto under ice cooling to stir for 30 minutes. To the reaction solution, benzyl bromide (519 μl) was added thereto with stirring under ice cooling, and then stirred for 4 hours at room temperature under argon atmosphere. To the reaction solution, methanol (1ml) and chloroform (15ml) were added under ice cooling, washed with water, and dried over anhydrous sodium sulfate, and then the solvent was distilled out in vacuo. The residue was purified by silica-gel chromatography (ethyl acetate : hexane = 1 : 3), To the reaction product in methylene chloride (5 ml), 10% trifluoro acetic acid (5.0ml) was added to stir for an hour under ice cooling, and then the solvent was distilled out in vacuo. The residue was purified by silica-gel chromatography (ethyl acetate : hexane = 1 : 5). To a suspension of the reacting product and 1H-tetrazol (65mg) in a mixture of dichloromethane and acetonitoril (1 : 1, 8ml), dibenzyloxy (diisopropylamino) phosphine (430mg) was added to stir for 12 hours at room temperature under argon atmosphere. Thereafter, water (15ml), ruthenium chloride (1mg) and sodium periodide (266mg) were added thereto to stir for 12 hours. To the reaction solution, methylene chloride (20ml) was added to obtain an organic layer, dried over anhydrous sodium sulfate, and then the solvent was distilled out in vacuo. The residue was purified by silica-gel chromatography (methyl acetate : hexane = 1 : 5). To the reaction product in methanol (4ml), water (0.5ml) and 10%-palladium carbon (80mg) were added to stir for 30 minutes at room temperature under argon The pH of the solution was adjusted to 7.0 by adding 0.1N sodium hydroxide solution to stir for 24 hours at room temperature under hydrogen atmosphere. The resulting solution was filtrated with celite (Johns Manville Sales Corp.), and then the solvent was distilled out in vacuo. The residue was dissolved into water (5ml), treated a cation exchange resin [WK-10 $Na^+$ type "Diaion" (trademark), 1 x 5cm], and lyophilized to afford the title compound (145mg).

Mass spectrum ($FAB^+$) m/z :
417.1 $(M+H)^+$.

$^1$H-NMR (270MHz) spectrum ($CD_3OD$)δ ppm :
0.89 (6H, t, J=6.4Hz, $CH_3$),
1.1 - 1.5 (8H, m, $C\underline{H_2}C\underline{H_2}$),
1.6 - 1.7 (1H, m, $Pr_2C\underline{H}$),
3.53 (1H, t, J=5.9Hz, H5),
3.62 (1H, dd, J=5.9, 9.9Hz, $Pr_2CHC\underline{H}aHb$),
3.69 (1H, dd, J=7.9, 9.4Hz, H2),
3.7 - 3.8 (2H, m, H6,H6'),
3.8 - 3.9 (1H, dd, J=5.9, 9.9Hz, $Pr_2CHCHa\underline{H}b$),
4.01 (1H, ddd, J=9.4, 3.0, 8.4Hz, H3),
4.09 (1H, d, J=3.0Hz, H4),
4.24 (1H, d, J=7.9Hz, H1).

$^{13}$C-NMR (68MHz) spectrum ($CD_3OD$) δ ppm :
14.8 ($CH_3$),
20.9, 21.0, 34.7, 34.8 ($CH_2$),
39.2 ($Pr_2\underline{C}H$),
62.6 (C6),
70.0 (C4),
72.3 (C2),
73.9 ($Pr_2CH\underline{C}H_2$),
76.4 (C5),
78.0 (C3),
105.5 (C1).

Example 19

Tetrasodium 2-propylpentyl β-D-glucopyranoside 2,3-diphosphate

To 2-propylpentyl β-D-glucopyranoside (600mg) in tetrahydrofuran (2.4ml), benzylaldehyde dimethyl acetal (1.5ml) and conc. sulfuric acid (50 μl) were added to stir for 65 hours at room temperature. The reaction solution was filtrated after neutralization by adding sodium carbonate, and then the solvent was distilled out in vacuo. The residue was purified by silica-gel chromatography (chloroform : methanol = 10 : 1). By treating as described in Example 15 excepting that a part of resulting compound (200mg), 1H-tetrazol (110mg) and dibenzyloxy (diisopropylamino) phosphine (726mg) were employed, the title compound was obtained (258mg).

Mass spectrum (FAB $^+$) m/z :
541.1 $(M+H)^+$.

$^1$H-NMR (270MHz) spectrum ($D_2O$)δ ppm :
0.92 (6H, t, J=6.4Hz, $CH_3$),
1.2 - 1.5 (8H, m, $C\underline{H}_2C\underline{H}_2$),
1.6 - 1.8 (1H, m, $Pr_2C\underline{H}$),
3.5 - 3.6 (1H, m, H5),
3.61 (1H, t, J=8.4Hz, H4),
3.65 (1R, dd, J=5.9, 9.9Hz, $Pr_2CHC\underline{H}aHb$),
3.75 (1H, dd, J=6.9, 9.9Hz, H6),
3.79 (1H, dd, J=5.9, 9.9Hz, $Pr_2CHCHa\underline{H}b$),
3.82 (1H, dd, J=5.4, 9.9Hz, m, H6'),
3.96 (1H, dd, J=7.9, 8.4Hz, H2),
4.16 (1H, q, J=8.4Hz, H3),
4.62 (1H, d, 1=7.9Hz, H1).

$^{13}$C-NMR (68MHz) spectrum ($D_2O$) δ ppm :
15.8 ($CH_3$),
21.1, 21.2, 34.5, 34.6 ($CH_2$),
38.9 ($Pr_2\underline{C}H$),
63.0 (C6),
72.3 (C4),
76.1 ($Pr_2CH\underline{C}H_2$),
77.9 (C5),
78.5 (C2),
81.1 (C3).
104.1 (C1).

Example 20

Hexasodium 2-propylpentyl β-D-glucopyranoside 2,3,4-triphosphate

To 2-propylpentyl β-D-glucopyranoside (100mg) in tetrahydrofuran (2ml), trimethylamine (52.4 μl) and trityl chloride (105mg) were added to stir for 100 hours at room temperature. After distilling out the solvent in vacuo, the residue was purified by silica-gel chromatography (chloroform : methanol = 10 : 1). By treating as described in Example 15 except that the resulting compound, 1H-tetrazol (46mg) and dibenzyloxy (diisopropylamino) phosphine (302mg) were selecting, the title compound (147mg) was obtained.

Mass spectrum (FAB $^+$) m/z :
665.0 $(M+H)^+$.

$^1$H-NMR (270MHz) spectrum ($D_2O$) δ ppm :
0.92 (6H, t, J=6.4Hz, $CH_3$),
1.3 - 1.5 (8H, m, $C\underline{H}_2C\underline{H}_2$),
1.7 - 1.8 (1H, m, $Pr_2C\underline{H}$),
3.5 - 3.7 (1H, m, H5),
3.65 (1H, dd, J=6.4, 9.9Hz, $Pr_2CHC\underline{H}aHb$),
3.81 (1H, dd, J=6.4, 9.9Hz, $Pr_2CHCHa\underline{H}b$),
3.85 (1H, dd, J=2.0, 12.9Hz, H6),
3.95 (1H, dd, J=4.0, 12.9Hz, m, H6'),

4.04 (1H, dd, J=7.4, 9.4Hz, H2),
4.10 (1H, q, J=9.4Hz, H4),
4.31 (1H, q, J=9.4Hz, H3),
4.66 (1H, d, J=7.4Hz, H1).

$^{13}$C-NMR (68MHz) spectrum ($D_2O$) δ ppm :

15.8 ($CH_3$),
21.1, 21.2, 34.5, 34.6 ($CH_2$),
38.8 ($Pr_2\underline{C}H$),
62.8 (C6),
74.0 (C4),
76.1 ($Pr_2CH\underline{C}H_2$),
77.5 (C5),
78.5 (C2),
80.6 (C3),
104.1 (C1).

Example 21

Tetrasodium 2-propylpentyl 0-β-D-galactopyranosyl-(1→4)-β-D-glucopyranoside 3',4'-diphosphate

To 2-propylpentyl 0-β-D-galactopyranosyl-(1→4)-β-D-glucopyranoside (200mg) in a mixture of acetone and tetrahydrofuran (1 : 3, 80ml), conc. sulfuric acid (30 μl) was added to stir for 24 hours at room temperature. After neutralizing the reaction solution with triethylamine, the solvent was distilled out in vacuo after filtration. The residue was purified by silica-gel chromatography (methylene : acetone = 1 : 1). The reaction product was dissolved into N,N-dimethylformamide (2ml), and sodium hydride (106mg) was added thereto under ice cooling to stir for 30 minutes. To the reaction mixture, benzyl bromide (284 μl) was added to stir under ice cooling, which was further stirred for 4 hours at room temperature under argon atmosphere. To the reaction solution, methanol (1ml) and methylene chloride (10ml) were added under ice cooling, washed with water and dried over anhydrous sodium sulfate, and then the solvent was distilled out in vacuo. The residue was purified by silica-gel chromatography (ethyl acetate : hexane = 1 : 4). To the reaction product in methylene chloride (3ml), 90% trifluoro acetic acid (0.8ml) was added to stir for an hour under ice cooling, and then the solvent was distilled out in vacuo. The residue was purified by silica-gel chromatography (ethyl acetate : hexane (1 : 5). By treating as described in Example 15 except that the resulting product (180mg), 1H-tetrazol (84mg) and dibenzyloxy (diisopropylamino) phosphine (277mg) were selected, the title compound (120mg) was obtained.

Mass spectrum (FAB $^+$) m/z :

703.1 $(M+H)^+$.

$^1$H-NMR (270MHz) spectrum ($D_2O$) δ ppm :

glucose
H1 (4.36, 1H, d, J=7.9Hz),
H2 (3.29, 1H, dd, J=7.9, 8.9Hz),
H3 (3.5 - 3.7, 1H, m),
H4 (3.6 - 3.7, 1H, m),
H5 (3.4 - 3.6, 1H, m),
H6' (3.87, 1H, dd, J=4.5, 12.4Hz),
H6 (3.94, 1H, dd, J=2.5, 12.4Hz),

galactose
H1 (4.53, 1H, d, J=7.9Hz),
H2 (3.7 - 3.8, 1H, m),
H3 (4.15, 1H, dt, J=3.0, 8.9Hz),
H4 (4.57, 1H, d, J=3.0Hz),
H5 (3.7 - 3.8, 1H, m),
H6, H6' (3.7 - 3.9, 2H, m),

aglycon
$CH_3$ (0.90, 6H, t, J=6.4Hz),
$CH_2$ (1.2 - 1.4, 8H, m),
CH (1.6 - 1.7, 1H,m),
$OC\underline{H}aHb$ (3.4 - 3.6, 1H, m),
$OCHa\underline{H}b$ (3.7 - 3.9. 1H, m).

$^{13}$C-NMR (68MHz) spectrum ($D_2O$) δ ppm :

glucose
C1 (104.0),
C2 (74.2),
C3 (75.7),

| | |
|---|---|
| | C4 (80.2), |
| | C5 (75.9), |
| | C6 (61.4). |
| galactose | C1 (104.4), |
| | C2 (71.8), |
| | C3 (76.2), |
| | C4 (71.7), |
| | C5 (75.5), |
| | C6 (60.5), |
| aglycon | $CH_3$ (14.6), |
| | $CH_2$ (20.4, 33.9), |
| | CH (38.3), |
| | $OCH_2$ (74.5). |

Example 22

Tetrasodium 2-tetradesylhexadesyl 0-β -D-galactopyranosyl-(1→4)-β-D-glucopyranoside 3',4'-diphosphate

By treating as described in example 21 except that 2-tetradesylhexadesyl 0-β-D-galactopyranosyl-(1→4)-β-D-glucopyranoside (150mg) was selected, the title compound (177mg) was obtained.

| | |
|---|---|
| Mass spectrum ($FAB^+$) m/z : | |
| | 1012.0 $(M+H)^+$. |
| $^1$H-NMR (270MHz) spectrum ($D_2O$) δ ppm : | |
| glucose | H1 (4.28, 1H, d, J=7.9Hz), |
| | H2 (3.26, 1H, t, J=7.9Hz), |
| | H3 (3.5 - 3.6, 1H, m), |
| | H4 (3.6 - 3.7, 1H, m), |
| | H5 (3.4 - 3.5, 1H, m), |
| | H6, H6' (3.8 - 3.9, 2H, m), |
| galactose | H1 (4.54, 1H, d, J=7.9Hz), |
| | H2 (3.6 - 3.8, 1H, m), |
| | H3 (4.1 - 4.2, 1H, m), |
| | H4 (4.5 - 4.7, 1H, m), |
| | H5 (3.7 - 3.8, 1H, m), |
| | H6, H6' (3.6 - 3.8, 2H, m), |
| aglycon | $CH_3$ (0.87, 6H, t, J=6.4Hz), |
| | $CH_2$ (1.2 - 1.4, 52H, m), |
| | CH (1.5 - 1.7, 1H, m), |
| | OC<u>H</u>aHb (3.3 - 3.4, 1H, m), |
| | OCHa<u>H</u>b (3.7 - 3.8, 1H, m). |
| $^{13}$C-NMR (68MHz) spectrum ($D_2O$) δ ppm : | |
| glucose | C1 (103.9), |
| | C2 (74.0), |
| | C3 (75.5), |
| | C4 (79.9), |
| | C5 (75.6), |
| | C6 (61.4), |
| galactose | C1 (104.0), |
| | C2 (71.3), |
| | C3 (76.4), |
| | C4 (71.7), |
| | C5 (75.2), |
| | C6 (60.8), |
| aglycon | $CH_3$ (14.5), |
| | $CH_2$ (23.3, 27.1, 30.2, 30.4, 30.5, 30.6, 31.1, 32.7), |
| | CH (38.7), |
| | $OCH_2$ (74.3). |

Example 23

Sodium 2-propylpentyl 0-α-L-fucopyranosyl-(1→2)-β-D-galactopyranoside 3-sulfate (23-1) and disodium 2-propylpentyl 0-α-L-fucopyranosyl-(1→2)-β-D-galactopyranoside 3-phosphate (23-2)

To a suspension of 2-propylpentyl-β-D-galactopyranoside (15.0g) and benzylaldehyde methyl acetal (15.6ml) in tetrahydrofuran (200ml), conc. sulfuric acid (1ml) was added to stir for 20 hours at room temperature. The reaction solution was neutralized by adding sodium carbonate, filtrated, and then the solvent was distilled out in vacuo. The residue was purified by silica-gel chromatography (ethyl acetate : hexane = 1 : 2). The reaction product and di-n-butyl-tin oxide (5.89g) in methanol (225ml) was refluxed for hours, and then the solvent was distilled out in vacuo. To the residue in tetrahydrofuran (100ml), tetra-n-butylammonium iodide (13.1g) and 4-methoxibenzochloride (4.81ml) were added to reflux for 4 hours, filtrated, and then the solvent was distilled out in vacuo. The residue was purified by silica-gel chromatography (ethyl acetate : hexane = 1 : 3). A part of reaction product (2.50g) and phenyl 2,3,4-tri-0-benzyl-1-thio-β-L-fucopyranoside (5.26g) which was separately prepared, were dissolved into a mixture of toluene and methylene chloride (3 : 1, 40ml), and Molecular Sieves 4A (Linde Co.) (12.5g) was added to stir for 24 hours under argon atmosphere. The reaction solution was cooled at -20°C, and N-iodosuccinicimide (4.49g) and trifluoromethane sulfonic acid (177 μl) were added to stir for an hour. The reaction solution was neutralized by adding triethylamine, filtrated, added toluene (100ml), washed with water, dried over anhydrous sodium sulfate, and then the solvent was distilled out in vacuo. The residue was purified by silica-gel chromatography (ethyl acetate : hexane = 1 : 3). To a part of the reaction product (540mg) in a mixture of methylene chloride and water (20 : 1, 5ml), DDQ (134mg) was added to stir for 2 hours at room temperature. The reaction solution was filtrated, and then the solvent was distilled out in vacuo. The residue was purified by silica-gel chromatography (ethyl acetate : hexane = 2 : 5) to afford 2-propylpentyl 0-(2,3,4-tri-0-benzyl α-L-fucopyranosyl-(1→ 2)-0-4,6-0-benzylidene-β-D-galactopyranoside (188mg).

To the resulting 2-propylpentyl 0-(2,3,4-tri-0-benzyl α-L-fucopyranosyl-(1→2)-0-4,6-0-benzylidene β-D-galactopyranoside (87.6mg) in N,N-dimethylformamide (1 ml), a complex of sulfur trioxide and pyridine was added to stir for an hour at room temperature, and then methanol (2ml), tetrahydrofuran (2ml) and sodium methoxide (29.7mg) were added to stir for an hour, and then the solvent was distilled out in vacuo. The residue was purified by silica-gel chromatography (chloroform : methanol = 10 : 1). To the reaction product in a mixture of tetrahydrofuran and methanol (1 : 1, 2ml), 20%-palladium carbon was added to stir for 16 hours at room temperature under hydrogen atmosphere. The reacting solution was filtrated, and then the solvent was distilled out in vacuo. The residue was dissolved into water (2ml) and lyophilized to afford the title compound of sodium 2-propylpentyl 0-α-L-fucopyranosyl-(1→2)-β-D-galactopyranoside 3-sulfate (37.5mg) was obtained by freeze drying. Furthermore, by treating as described in Example 12 excepting that 2-propylpentyl 0-(2,3,4-tri-0-benzyl α-L-fucopyranosyl)-(1→2)-0-4,6-0-benzylidene-β-D-glucopyranoside (82.5mg) and dibenzyloxy (diisopropylamino) phosphine (71.5mg) were selected, the other title compound of disodium 2-propylpentyl 0-α-L-fucopyranosyl-(1→2)-β-D-galactopyranoside 3-phosphate (41.4mg) was obtained.

Compound 23 - 1

| | |
|---|---|
| Mass spectrum (FAB $^-$) m/z : | |
| | 517.2 $(M-Na)^-$. |
| $^1$H-NMR (270MHz) spectrum ($CD_3OD$)δ ppm : | |
| galactose | H1 (4.38, 1H, d, J=7.4Hz), |
| | H2 (3.89, 1H, dd, J=7.4, 9.4Hz), |
| | H3 (4.43, 1H, dd, J=9.4, 3.0Hz), |
| | H4 (4.25, 1H, d, J=3.0Hz), |
| | H5 (3.54, 1H, t, J=6.4Hz), |
| | H6, H6' (3.74, 2H, d, J=6.4Hz), |
| fucose | H1 (5.3 - 5.4, 1H, m), |
| | H2 (3.7 - 3.8, 1H, m), |
| | H3 (3.7 - 3.8, 1H, m), |
| | H4 (3.6 - 3.7, 1H, m), |
| | H5 (4.3 - 4.5, 1H, m), |
| | 6-$CH_3$ (1.18, 2H, d, J=6.4Hz), |
| aglycon | $CH_3$ (0.90, 6H, t, J=6.4Hz), |
| | $CH_2$ (1.2 - 1.4, 8H, m), |
| | CH (1.5 - 1.6, 1H,m), |
| | OC<u>H</u>aHb (3.41, 1H, dd, J=5.4, 9.9Hz), |
| | OCHa<u>H</u>b (3.82, 1H, dd, J=6.4, 9.9Hz). |
| $^{13}$C-NMR (68MHz) spectrum ($CD_3OD$) δ ppm : | |

galactose: C1 (103.9), C2 (74.0), C3 (82.8), C4 (68.9), C5 (76.2), C6 (62.3),
fucose: C1 (100.0), C2 (70.2), C3 (71.6), C4 (73.7), C5 (67.5), C6 (16.8).
aglycon: $CH_3$ (14.8), $CH_2$ (20.9, 21.1, 34.8, 34.9), CH (39.4), $OCH_2$ (73.8).

Compound 23 - 2

Mass spectrum (FAB $^+$) m/z :
563.2 $(M+H)^+$.

$^1$H-NMR (270MHz) spectrum ($CD_3OD$) $\delta$ ppm :
galactose: H1 (4.39, 1H, d, J=7.4Hz), H2 (3.7 - 3.9, 1H, m), H3 (4.2 - 4.3, 1H, m), H4 (4.1 - 4.2, 1H, m), H5 (3.5 - 3.7, 1H, m), H6, H6' (3.7 - 3.9, 2H, m),
fucose: H1 (5.52, 1H, m), H2 (3.7 - 3.9, 1H, m), H3 (3.7 - 3.9, 1H, m), H4 (3.6 - 3.7, 1H, m), H5 (4.3 - 4.5, 1H, m), 6-$CH_3$ (1.20, 2H, d, J=6.9Hz),
aglycon: $CH_3$ (0.90, 6H, t, J=6.4Hz), $CH_2$ (1.2 - 1.4, 8H, m), CH (1.5 - 1.7, 1H,m), OC<u>H</u>aHb (3.42, 1H, dd, J=5.4, 9.9Hz), OCHa<u>H</u>b (3.82, 1H, dd, J=6.4, 9.9Hz).

$^{13}$C-NMR (68MHz) spectrum ($CD_3OD$) $\delta$ ppm :
galactose: C1 (103.7), C2 (75.4), C3 (78.9), C4 (69.8), C5 (76.0), C6 (62.3),
fucose: C1 (99.8), C2 (69.5), C3 (71.4), C4 (73.6), C5 (67.3), C6 (16.8),
aglycon: $CH_3$ (14.8), $CH_2$ (20.7, 20.9, 34.5, 34.6), CH (39.2), $OCH_2$ (73.8).

Example 24

Disodium 2-propylpentyl 0-α-L-fucopyranosyl-(1→2)-β-D-galactopyranoside 3,3'-disulfate (24 - 1) and tetrasodium 2-propylpentyl 0-α-L-fucopyranosyl-(1→2)-β-D-galactopyranoside 3,3'-diphosphate (24 - 2)

By treating as described in Example 23 except that 2-propylpentyl 4,6-0-benzylidene-3-0-(4-methoxybenzyl)-β-D-galactopyranoside (630mg) and phenyl 2,4-di-0-benzyl-3-0-(4-methox ybenzyl)-1-thio-β-L-fucopyranoside (840mg) were selected as a starting compound were selected, 2-propylpentyl 0-(2,4-di-0-benzyl α-L-fucopyranosyl)-(1→2)-0-4,6-0-benzylidene-β -D-galactopyranoside (327mg) was obtained. By treating as described in Example 23 excepting that the resulting compound (150mg) and a complex of sulfur trioxide and pyridine (167mg) were selected, the title compound of disodium 2-propylpentyl 0-α-L-fucopyranosyl-(1→2)-β-D-galactopyranoside 3,3'-disulfate (112mg) was obtained. Furthermore, by treating as described in Example 12 excepting that 2-propylpentyl 0-(2,4-di-0-benzyl-α-L-fucopyranosyl)-(1→2)-0-4,6-0-benzylidene-β-D-galactopyranoside (150mg) and dibenzyloxy (diisopropylamino) phosphine (293mg) were selected as a starting compound were selected, the other title compound of tetrasodium 2-propylpentyl 0-α-L-fucopyranosyl-(1→2)-β-D-galactopyranoside 3,3'-diphosphate (91.3mg) was obtained.

Compound 24 - 1

Mass spectrum (FAB $^-$) m/z :

| | |
|---|---|
| | 619.1 (M-Na) $^-$ . |
| $^1$H-NMR (270MHz) spectrum ($CD_3OD$) δ ppm : | |
| galactose | H1 (4.41, 1H, d, J=6.9Hz), |
| | H2 (3.93, 1H, dd, J=6.9, 9.9Hz), |
| | H3 (4.46, 1H, dd, J=9.9, 3.0Hz), |
| | H4 (4.29, 1H, d, J=3.0Hz), |
| | H5 (3.54, 1H, t, J=5.9Hz), |
| | H6, H6' (3.74, 2H, d, J=5.9Hz), |
| fucose | H1 (5.36, 1H, d, J=4.0Hz), |
| | H2 (3.95, 1H, dd, J=4.0, 9.9Hz), |
| | H3 (4.49, 1H, dd, J=9.9, 3.0Hz), |
| | H4 (4.05, 1H, d, J=3.0Hz), |
| | H5 (4.4 - 4.5, 1H, m), |
| | 6-$CH_3$ (1.19, 2H, d, J=6.4Hz), |
| aglycon | $CH_3$ (0.90, 6H, t, J=6.4Hz), |
| | $CH_2$ (1.2-1.5, 8H, m), |
| | CH (1.5-1.7, 1H,m), |
| | OC<u>H</u>aHb (3.45, 1H, dd, J=4.9, 9.4Hz), |
| | OCHa<u>H</u>b (3.80, 1H, dd, J=6.9, 9.4Hz). |
| $^{13}$C-NMR (68MHz) spectrum ($CD_3OD$)δ ppm : | |
| galactose | C1 (103.7), |
| | C2 (73.9), |
| | C3 (82.8), |
| | C4 (68.9), |
| | C5 (76.2), |
| | C6 (62.3), |
| fucose | C1 (100.3), |
| | C2 (68.0), |
| | C3 (79.2), |
| | C4 (72.0), |
| | C5 (67.4), |
| | C6 (16.8), |
| aglycon | $CH_3$ (14.9), |
| | $CH_2$ (20.8, 21.1, 34.7, 34.9), |
| | CH (39.4), |
| | $OCH_2$ (73.8). |

Compound 24 - 2

Mass spectrum (FAB$^+$) m/z :

687.1 $(M+H)^+$.

$^1$H-NMR (270MHz) spectrum ($CD_3OD$) δ ppm :

| | |
|---|---|
| galactose | H1 (4.43, 1H, d, J=7.9Hz), |
| | H2 (3.7 - 3.8, 1H, m), |
| | H3 (4.2 - 4.3, 1H, m), |
| | H4 (4.1 - 4.2, 1H, m), |
| | H5 (3.5 - 3.7, 1H, m), |
| | H6, H6' (3.7 - 3.9, 2H, m), |
| fucsoe | H1 (5.43, 1H, d, J=4.0Hz), |
| | H2 (3.9 - 4.0, 1H, m), |
| | H3 (4.2 - 4.3, 1H, m), |
| | H4 (3.9 - 4.0, 1H, m), |
| | H5 (4.3 - 4.4, 1H, m), |
| | 6-$CH_3$ (1.20, 2H, d, J=6.4Hz), |
| aglycon | $CH_3$ (0.87, 6H, t, J=6.9Hz), |
| | $CH_2$ (1.1 - 1.5, 8H, m), |
| | CH (1.5 - 1.7, 1H, m), |
| | OC<u>H</u>aHb (3.50, 1H, dd, J=5.4, 9.9Hz), |
| | OCHa<u>H</u>b (3.78, 1H, dd, J=6.4, 9.9Hz). |

$^{13}$C-NMR (68MHz) spectrum ($CD_3OD$) δ ppm :

| | |
|---|---|
| galactose | C1 (103.1), |
| | C2 (76.0), |
| | C3 (78.5), |
| | C4 (69.4), |
| | C5 (75.4), |
| | C6 (62.0), |
| fucose | C1 (99.9), |
| | C2 (68.7), |
| | C3 (74.6), |
| | C4 (72.4), |
| | C5 (67.1), |
| | C6 (16.8), |
| aglycon | $CH_3$ (14.7), |
| | $CH_2$ (20.2, 20.5, 33.9, 34.1), |
| | CH (38.7), |
| | $OCH_2$ (74.5). |

## Example 25

### Disodium 2-propylpentyl 0-α-L-fucopyranosyl-(1→2)-β-D-galactopyranoside 3,6-disulfate

By treating as described in Example 2 except that sodium 2-propylpentyl 0-α-L-fucopyranosyl-(1→2)-β-D-galactopyranoside 3-sulfate (20.0mg) obtained by Example 23 and a complex of sulfurous acid and trimethyl ammonium (6.2mg) were selected, the title compound (12.1mg) was obtained.

Mass spectrum ($FAB^-$) m/z :

619.1 $(M-Na)^-$.

$^1$H-NMR (270MHz) spectrum (DMSO-$d_6$) δ ppm :

| | |
|---|---|
| galactose | H1 (4.39, 1H, d, J=7.4Hz), |
| | H2 (3.89, 1H, dd, J=7.4, 9.4Hz), |
| | H3 (4.44, 1H, dd, J=9.4, 3.0Hz), |
| | H4 (4.30, 1H, d, J=3.0Hz), |
| | H5 (3.75, 1H, t, J=6.4Hz), |
| | H6, H6' (4.06, 2H, d, J=6.4Hz), |
| fucose | H1 (5.3 - 5.4, 1H, m), |
| | H2 (3.7 - 3.8, 1H, m), |
| | H3 (3.7 - 3.8, 1H, m), |
| | H4 (3.6 - 3.7, 1H, m), |
| | H5 (4.3 - 4.5, 1H, m), |

| | |
|---|---|
| | 6-$CH_3$ (1.18, 2H, d, J=6.4Hz), |
| aglycon | $CH_3$ (0.90, 6H, t, J=6.4Hz), |
| | $CH_2$ (1.2 - 1.4, 8H, m), |
| | CH (1.5 - 1.6, 1H, m), |
| | OC<u>H</u>aHb (3.4 - 3.5, 1H, m), |
| | OCHa<u>H</u>b (3.8 - 3.9, 1H, m). |
| $^{13}$C-NMR (68MHz) spectrum (DMSO-$d_6$) δ ppm : | |
| galactose | C1 (104.1), |
| | C2 (73.9), |
| | C3 (83.0), |
| | C4 (69.2), |
| | C5 (74.1), |
| | C6 (66.7), |
| fucose | C1 (100.2), |
| | C2 (70.3), |
| | C3 (71.8), |
| | C4 (73.7), |
| | C5 (67.5), |
| | C6 (16.8), |
| aglycon | $CH_3$ (14.8), |
| | $CH_2$ (21.0, 21.1, 34.8, 35.0), |
| | CH (39.4), |
| | $OCH_2$ (73.9). |

<u>Example 26</u>

<u>Trisodium 2-propylpentyl 0-α-L-fucopyranosyl-(1→2)-β-D-galactopyranoside 3,3',6-trisulfate</u>

By treating as described in Example 2 except that disodium 2-propylpentyl 0-α-L-fucopyranosyl-(1→2)-β-D-galactopyranoside 3,3'-disulfate (40.0mg) obtained by the Example 24 and a complex of sulfurous acid and trimethyl ammonium (10.4mg) were selected, the title compound (25.1mg) was obtained.

| | |
|---|---|
| Mass spectrum (FAB$^-$) m/z : | |
| | 721.0 (M-Na)$^-$. |
| $^1$H-NMR (270MHz) spectrum (DMSO-$d_6$) δ ppm : | |
| galactose | H1 (4.41, 1H, d, J=7.8Hz), |
| | H2 (3.89, 1H, dd, J=7.8, 9.4Hz), |
| | H3 (4.45, 1H, dd, J=9.4, 3.0Hz), |
| | H4 (4.30, 1H, d, J=3.0Hz), |
| | H5 (3.77, 1H, t, J=6.8Hz), |
| | H6, H6' (4.07, 2H, d, J=6.8Hz), |
| fucose | H1 (5.39, 1H, d, J=4.0Hz), |
| | H2 (3.94, 1H, dd, J=4.0, 9.9Hz), |
| | H3 (4.49, 1H, dd, J=9.9, 3.0Hz), |
| | H4 (4.06, 1H, d, J=3.0Hz), |
| | H5 (4.4 - 4.5, 1H, m), |
| | 6-$CH_3$ (1.19, 2H, d, J=6.4Hz), |
| aglycon | $CH_3$ (0.90, 6H, t, J=6.4Hz), |
| | $CH_2$ (1.2 - 1.4, 8H, m), |
| | CH (1.5 - 1.6, 1H, m), |
| | OC<u>H</u>aHb (3.4 - 3.5, 1H, m), |
| | OCHa<u>H</u>b (3.8 -3.9, 1H, m). |
| $^{13}$C-NMR (68MHz) spectrum (DMSO-$d_6$) δ ppm : | |
| galactose | C1 (104.2), |
| | C2 (74.0), |
| | C3 (82.7), |
| | C4 (69.3), |
| | C5 (74.1), |
| | C6 (66.7), |

| | |
|---|---|
| fucose | C1 (100.4), |
| | C2 (68.1), |
| | C3 (79.4), |
| | C4 (72.1), |
| | C5 (67.5), |
| | C6 (16.9), |
| aglycon | $CH_3$ (14.9), |
| | $CH_2$ (21.0, 21.1, 34.8, 34.9), |
| | CH (39.4), |
| | $OCH_2$ (73.9). |

Example 27

Sodium 2-tetradesylhexadesyl 0-α-L-fucopyranosyl-(1→2)-β-D-galactopyranoside 3-sulfate (27 - 1) and disodium 2-tetradesylhexadesyl 0-α-L-fucopyranosyl-(1→2)-β-D-galactopyranoside 3-phosphate (27 - 2)

By treating as described in Example 23 except that 2-tetradesylhexadesyl β-D-galactopyranoside (6.5g) was selected as a starting compound, the title compound of sodium 2-tetradesylhexadesyl 0-α-L-fucopyranosyl-(1→2)-β-D-galactopyranoside 3-sulfate (50.0mg) and disodium 2-tetradesylhexadesyl 0-α-L-fucopyranosyl-(1→2)-β-D-galactopyranoside 3-phosphate (60.8mg) were obtained.

Compound 27 - 1

| | |
|---|---|
| Mass spectrum (FAB $^-$ ) m/z : | 825.5 $(M-Na)^-$. |
| $^1H$-NMR (270MHz) spectrum ($CD_3OD$) δ ppm : | |
| galactose | H1 (4.4 - 4.5, 1H, m), |
| | H2 (3.8 - 3.9, 1H, m), |
| | H3 (4.4 - 4.5, 1H, m), |
| | H4 (4.33, 1H, d, J=4.0Hz), |
| | H5 (3.6 - 3.7, 1H, m), |
| | H6, H6' (3.7 - 3.9, 2H, m), |
| fucose | H1 (5.2 - 5.3, 1H, m), |
| | H2 (3.7 - 3.8, 1H, m), |
| | H3 (3.7 - 3.8, 1H, m), |
| | H4 (3.6 - 3.8, 1H, m), |
| | H5 (4.3 - 4.4, 1H, m), |
| | 6-$CH_3$ (1.18, 2H, d, J=6.4Hz), |
| aglycon | $CH_3$ (0.90, 6H, t, J=6.9Hz), |
| | $CH_2$ (1.3 - 1.4, 8H, m), |
| | CH (1.5 - 1.7, 1H,m), |
| | OC<u>H</u>aHb (3.4 - 3.5, 1H, m), |
| | OCHa<u>H</u>b (3.7 - 3.8, 1H, m). |
| $^{13}C$-NMR (68MHz) spectrum ($CD_3OD$)δ ppm : | |
| galactose | C1 (103.2), |
| | C2 (74.6), |
| | C3 (82.5), |
| | C4 (67.9), |
| | C5 (75.0), |
| | C6 (61.2), |
| fucose | C1 (100.0), |
| | C2 (69.7), |
| | C3 (71.3), |
| | C4 (74.4), |
| | C5 (67.5), |
| | C6 (16.9), |
| aglycon | $CH_3$ (14.7), |
| | $CH_2$ (23.6, 27.2, 27.5, 30.4, 30.6, 30.7, 30.8, 30.9, 31.7, 32.9), |
| | CH (39.3), |
| | $OCH_2$ (74.2). |

Compound 27 - 2

Mass spectrum ($FAB^+$) m/z : 871.5 $(M+H)^+$.

$^1H$-NMR (270MHz) spectrum ($D_2O$) $\delta$ ppm :

| | |
|---|---|
| galactose | H1 (4.3 - 4.5, 1H, m), |
| | H2 (3.6 - 3.8, 1H, m). |
| | H3 (4.2 - 4.3, 1H, m), |
| | H4 (4.1 - 4.2, 1H, m), |
| | H5 (3.5 - 3.6, 1H, m), |
| | H6, H6' (3.6 - 3.8, 2H, m), |
| fucose | H1 (5.3 - 5.4, 1H, m), |
| | H2 (3.7 - 3.8, 1H, m), |
| | H3 (3.8 - 3.9, 1H, m), |
| | H4 (3.6 - 3.8, 1H, m), |
| | H5 (4.2 - 4.3, 1H, m), |
| | 6-$CH_3$ (1.14, 2H, d, J=6.4Hz), |
| aglycon | $CH_3$ (0.84, 6H, t, J=6.9Hz), |
| | $CH_2$ (1.1 - 1.4, 8H, m), |
| | CH (1.4 - 1.6, 1H,m), |
| | OC<u>H</u>aHb (3.3 - 3.4, 1H, m), |
| | OCHa<u>H</u>b (3.6 - 3.7, 1H, m). |

$^{13}C$-NMR (68MHz) spectrum ($D_2O$) $\delta$ ppm :

| | |
|---|---|
| galactose | C1 (103.0), |
| | C2 (75.4), |
| | C3 (78.9), |
| | C4 (68.6), |
| | C5 (75.0), |
| | C6 (61.2), |
| fucose | C1 (99.7), |
| | C2 (69.2), |
| | C3 (71.0), |
| | C4 (73.2), |
| | C5 (67.1), |
| | C6 (16.9), |
| aglycon | $CH_3$ (14.6), |
| | $CH_2$ (23.4, 27.0, 27.3, 30.2, 30.4, 30.5, 30.6, 30.7, 31.4, 32.8), |
| | CH (39.2), |
| | $OCH_2$ (73.8). |

Example 28

Disodium -2-tradesylhexadesyl 0-α-L-fucopyranosyl-(1→2)-β-D-galactopyranoside 3,3'-disulfate (28-1) and tetrasodium 2-tetradesylhexadesyl 0-α-L-fucopyranosyl-(1→2)-β -D-galactonyranoside 3,3'-diphosphate (28 - 2)

By treating as described in Example 24 except that 2-tetradesylhexadesylβ-D-galactopyranoside (6.5g) was selected, the title compound of disodium 2-tetradesylhexadesyl 0-α-L-fucopyranosyl-(1→2)-β-D-galactopyranoside 3,3'-disulfate (78.0mg) and tetrasodium 2-tetradesylhexadesyl 0-α-L-fucopyranosyl-(1→ 2)-β-D-galactopyranoside 3,3'-diphosphate (11.2mg) were obtained.

Compound 28 - 1

Mass spectrum ($FAB^-$) m/z : 927.5 $(M-Na)^-$.

$^1H$-NMR (270MHz) spectrum ($CD_3OD$) $\delta$ ppm :

| | |
|---|---|
| galactose | H1 (4.5 - 4.6, 1H, m), |
| | H2 (3.7 - 3.8, 1H, m), |
| | H3 (4.5 - 4.6, 1H, m), |
| | H4 (4.3 - 4.4, 1H, m), |

H5 (3.6 - 3.7, 1H, m),
H6, H6' (3.6 - 3.8, 2H, m),

fucose
H1 (5.2 - 5.3, 1H, m),
H2 (3.9 - 4.0, 1H, m),
H3 (4.5 - 4.6, 1H, m),
H4 (4.0 - 4.2, 1H, m),
H5 (4.3 - 4.4, 1H, m),
6-$CH_3$ (1.22, 2H, d, J=6.4Hz),

aglycon
$CH_3$ (0.89, 6H, t, J=6.4Hz),
$CH_2$ (1.2 - 1.4, 8H, m),
CH (1.5 - 1.7, 1H, m),
OC<u>H</u>aHb (3.4 - 3.5, 1H, m),
OCHa<u>H</u>b (3.6 - 3.7, 1H, m).

$^{13}$C-NMR (68MHz) spectrum ($CD_3OD$) δ ppm :

galactose
C1 (103.0),
C2 (75.4),
C3 (82.3),
C4 (67.6),
C5 (74.7),
C6 (60.9),

fucose
C1 (100.5),
C2 (67.5),
C3 (79.0),
C4 (71.4),
C5 (67.4),
C6 (16.7).

aglycon
$CH_3$ (14.7),
$CH_2$ (23.6, 26.9, 27.3, 30.3, 30.5, 30.6, 30.7, 31.4, 31.7, 32.9),
CH (39.2).
$OCH_2$ (74.3).

<u>Compound 28 - 2</u>

Mass spectrum (FAB $^+$) m/z :
995.5 (M+R) $^+$.

$^1$H-NMR (270MHz) spectrum ($D_2O$)δ ppm :

galactose
H1 (4.5 - 4.6, 1H, m),
H2 (3.9 - 4.1, 1H, m),
H3 (4.0 - 4.1, 1H, m),
H4 (3.9 - 4.0, 1H, m),
H5 (3.5 - 3.7, 1H, m),
H6, H6' (3.6 - 3.8, 2H, m),

fucose
H1 (4.8 - 4.9, 1H, m),
H2 (3.6 - 3.7, 1H, m),
H3 (4.2 - 4.3, 1H, m),
H4 (3.9 - 4.1, 1H, m),
H5 (4.1 - 4.3, 1H, m),
6-$CH_3$ (1.30, 2H, d, J=6.4Hz),

aglycon
$CH_3$ (0.89, 6H, t, J=6.4Hz),
$CH_2$ (1.2 - 1.4, 8H, m),
CH (1.5 - 1.7, 1H,m),
OC<u>H</u>aHb (3.4 - 3.5, 1H, m),
OCHa<u>H</u>b (3.6 - 3.7, 1H, m).

$^{13}$C-NMR (68MHz) spectrum ($D_2O$) δ ppm :

galactose
C1 (103.7),
C2 (74.5),
C3 (78.4),
C4 (71.3),
C5 (74.4),

| | |
|---|---|
| | C6 (60.7), |
| fucose | C1 (100.7), |
| | C2 (71.2), |
| | C3 (76.2), |
| | C4 (71.5), |
| | C5 (68.1), |
| | C6 (16.6), |
| aglycon | $CH_3$ (14.5), |
| | $CH_2$ (23.4, 26.9, 27.1, 30.2, 30.3, 30.4, 30.6, 31.1, 31.2, 32.7), |
| | CH (38.8), |
| | $OCH_2$ (73.6). |

Example 29

Disodium 2-tetradesylhexadesyl 0-α-L-fucopyranosyl-(1→2)-β -D-galactopyranoside 3,6-disulfate

By treating as described in Example 2 except that sodium 2-tetradesylhexadesyl 0-α-L-fucopyranosyl-(1→2)-β-D-galactopyranoside 3-sulfate (20.0mg) obtained by Example 27 and a complex of sulfurous acid and trimethyl ammonium (4.0mg) were selected, the title compound (10.1mg) was obtained.

| | |
|---|---|
| Mass spectrum ($FAB^-$) m/z : | |
| | 927.5 $(M-Na)^-$. |
| $^1$H-NMR (270MHz) spectrum ($CD_3OD$) δ ppm : | |
| galactose | H1 (4.4 - 4.5, 1H, m), |
| | H2 (3.8 - 3.9, 1H, m), |
| | H3 (4.4 - 4.5, 1H, m), |
| | H4 (4.33, 1H, d, J=4.0Hz), |
| | H5 (3.8 - 3.9, 1H, m), |
| | H6, H6' (4.0 - 4.2, 2H, m), |
| fucose | H1 (5.2 - 5.3, 1H, m), |
| | H2 (3.7 - 3.8, 1H, m), |
| | H3 (3.7 - 3.8, 1H, m), |
| | H4 (3.6 - 3.8, 1H, m), |
| | H5 (4.3 - 4.4, 1H, m), |
| | 6-$CH_3$ (1.18, 2H, d, J=6.4Hz), |
| aglycon | $CH_3$ (0.90, 6H, t, J=6.9Hz), |
| | $CH_2$ (1.3 - 1.4, 8H, m), |
| | CH (1.5 - 1.7, 1H, m), |
| | OC<u>H</u>aHb (3.4 - 3.5, 1H, m), |
| | OCHa<u>H</u>b (3.7 - 3.8, 1H, m). |
| $^{13}$C-NMR (68MHz) spectrum ($CD_3OD$) δ ppm : | |
| galactose | C1 (103.3), |
| | C2 (74.6), |
| | C3 (82.6), |
| | C4 (68.1), |
| | C5 (72.9), |
| | C6 (65.7), |
| fucose | C1 (100.1), |
| | C2 (69.9), |
| | C3 (71.3), |
| | C4 (74.4), |
| | C5 (67.5), |
| | C6 (16.8), |
| aglycon | $CH_3$ (14.7), |
| | $CH_2$ (23.6, 27.4, 27.5, 30.5, 30.6, 30.7, 30.8, 30.9, 31.7, 32.9), |
| | CH (39.3), |
| | $OCH_2$ (74.2). |

Example 30

Trisodium 2-tetradesylhexadesyl 0-α-L-fucopyranosyl-(1→2)-β-D-galactopyranoside 3,3',6-trisulfate

By treating as described in Example 2 except that disodium 2-tetradesylhexadesyl 0-α-L-fucopyranosyl-(1→2)-β-D-galactopyranoside 3,3'-disulfate (40.0mg) obtained by the Example 28 and a complex of sulfurous acid and trimethyl ammonium (7.1mg) were selected, the title compound (23.2mg) was obtained.

Mass spectrum ($FAB^-$) m/z :
1029.4 $(M-Na)^-$.
$^1$H-HMR (270MHz) spectrum ($CD_3OD$) δ ppm :

| | |
|---|---|
| galactose | H1 (4.4 - 4.5, 1H, m), |
| | H2 (3.8 - 3.9, 1H, m), |
| | H3 (4.4 - 4.5, 1H, m), |
| | H4 (4.3 - 4.4, 1H, m), |
| | H5 (3.8 - 3.9, 1H, m), |
| | H6, H6' (3.9 - 4.2, 2H, m), |
| fucose | H1 (5.2 - 5.3, 1H, m), |
| | H2 (3.9 - 4.0, 1H, m), |
| | H3 (4.5 - 4.6, 1H, m), |
| | H4 (4.0 - 4.2, 1H, m), |
| | H5 (4.3 - 4.4, 1H, m), |
| | 6-$CH_3$ (1.20, 2H, d, J=6.4Hz), |
| aglycon | $CH_3$ (0.89, 6H, t, J=6.9Hz), |
| | $CH_2$ (1.3 - 1.4, 8H, m), |
| | CH (1.5 - 1.7, 1H, m), |
| | OC<u>H</u>aHb (3.4 - 3.5, 1H, m), |
| | OCHa<u>H</u>b (3. 7 - 3. 8, 1H, m). |

$^{13}$C-NMR (68MHz) spectrum ($CD_3OD$) δ ppm :

| | |
|---|---|
| galactose | C1 (103.2), |
| | C2 (74.7), |
| | C3 (82.6), |
| | C4 (68.3), |
| | C5 (72.9), |
| | C6 (65.9), |
| fucose | C1 (100.4), |
| | C2 (67.5), |
| | C3 (79.1), |
| | C4 (71.6), |
| | C5 (67.4), |
| | C6 (16.8), |
| aglycon | $CH_3$ (14.8), |
| | $CH_2$ (23.5, 27.4, 27.5, 30.4, 30.6, 30.7, 30.8, 30.9, 31.7, 32.9), |
| | CH (39.3), |
| | $OCH_2$ (74.3). |

Example 31

Sodium 2-tetradesylhexadesyl β-D-galactopyranoside 3-sulfate

By treating as described in Example 1 except that 2-tetradesylhexadesyl β-D-galactopyranoside (250mg) and a complex of sulfurous acid and trimethyl ammonium (69.5mg) were selected, the title compound (198mg) was obtained.

Mass spectrum ($FAB^-$) m/z :
679.5 $(M-Na)^-$, 701.5 $(M-H)^-$.
$^1$H-NMR (270MHz) spectrum ($CD_3OD$) δ ppm :
0.90 (6H, t, J=6.8Hz, $CH_3$),
1.3 (52H, s, CH2),
1.5 - 1.7 (1H, m, $(C_{14}H_{29})_2$(C<u>H</u>),

3.42 (1H, dd, J=6.4, 9.8Hz, $(C_{14}H_{29})_2(CHC\underline{H}aHb)$,
3.53 (1H, t, J=6.4Hz, H5),
3.70 (1H, dd, J=7.8, 9.3Hz, H2),
3.74 (2H, d, J=6.4Hz, H6, H6'),
3.80 (1H, dd, J=6.4, 9.3Hz, $(C_{14}R_{29})_2CHCHa\underline{H}b$),
4.21 (1H, dd, J=3.4, 9.3Hz, H3),
4.25 (1H, d, J=3.4Hz, H4),
4.28 (1H, d, J=7.8Hz, H1).

$^{13}C$-NMR (68MHz) spectrum ($CD_3OD$) $\delta$ ppm :

14.4 ($CH_3$),
23.6, 27.6, 27.7, 30.3, 30.6, 31.0, 32.0, 32.9 ($CH_2$),
39.4 (CH),
62.2 (C6),
68.5 (C4),
70.7 (C2),
74.0 ($O\underline{C}H_2$),
76.5 (C5),
82.2 (C3),
105.0 (C1),

Example 32

Disodium 2-tetradesylhexadesyl β-D-galactopyranoside 3,6-disulfate

By treating as described in Example 1 except that 2-tetradesylhexadesyl β-D-galactopyranoside (250mg) and a complex of sulfurous acid and trimethyl ammonium (127mg) were selected, the title compound (221mg) was obtained.

Mass spectrum ($FAB^-$) m/z :

781.4 $(M-Na)^-$ , 803.4 $(M-H)^-$.

$^1H$-HMR (270MHz) spectrum ($CD_3OD$ - $D_2O$) $\delta$ ppm :

0.90 (6H, t, J=6.8Hz, $CH_3$),
1.30 (52H, s, $C\underline{H}_2$),
1.5 - 1.7 (1H, m, $(C_{14}H_{29})_2C\underline{H}$),
3.41 (1H, dd, J=5.9, 9.8Hz, $(C_{14}R_{29})_2CHC\underline{H}aHb$),
3.70 (1H, dd, J=7.8, 9.3Hz, H2),
3.78 (1H, dd, J=6.4, 9.8Hz, $(C_{14}R_{29})_2CHCHa\underline{H}b$),
3.82 (1H, dd, J=6.4, 6.1Hz, H5),
4.16 (1H, dd, J=6.4, 10.3Hz, H6),
4.22 (1H, dd, J=6.1, 10.3Hz, H6'),
4.25 (1H, d, J=9.3Hz, H3),
4.27 (1H, s, H4),
4.30 (1H, d, J=7.8Hz, H1).

$^{13}C$-NMR (68MHz) spectrum ($CD_3OD$ - $H_2O$) $\delta$ ppm :

14.7 ($CH_3$),
23.6, 27.2, 30.4, 30.6, 30.8, 30.9, 31.1, 32.9 ($CH_2$),
38.8 (CH),
66.3 (C6),
67.2 (C4),
69.9 (C2),
72.6 (C5),
74.6 ($OCH_2$),
81.3 (C3),
104.3 (C1).

Example 33

Trisodium 2-tetradesylhexadesyl β-D-galactopyranoside 3,4,6-trisulfate (33 - 1) and tetrasodium 2-tetradesylhexadesyl β-D-galactopyranoside 2, 3, 4, 6-tetrasulfate (33 - 2)

By treating as described in Example 2 except that sodium 2-tetradesylhexadesyl β-D-galactopyranoside 3-sulfate (100mg) obtained by Example 31 and a complex of sulfurous acid and trimethyl ammonium (105mg) were selected, the title compound of trisodium 2-tetradesylhexadesyl β-D-galactopyranoside 3,4,6-trisulfate (57mg) and tetrasodium 2-tetradesylhexadesyl β-D-galactopyranoside 2,3,4,6 -tetrasulfate (41mg) were obtained.

Compound 33 - 1

Mass spectrum ($FAB^-$) m/z :
883.4 $(M-Na)^-$, 905.4 $(M-H)^-$.

$^1$H-NMR (270MHz) spectrum ($CD_3OD$ - $D_2O$) δ ppm :
0.84 (6H, t, J=6.8Hz, $CH_3$),
1.30 (52H, s, $C\underline{H}_2$),
1.5 - 1.7 (1H, m, $(C_{14}H_{29})_2C\underline{H}$),
3.42 (1H, dd, J=5.9, 9.3Hz, $(C_{14}H_{29})_2CHC\underline{H}aHb$),
3.57 (1H, dd, J=8.4, 9.4Hz, H2),
3.78 (1H, dd, J=6.4, 9.3Hz, $(C_{14}H_{29})_2CHCHa\underline{H}b$),
4.00 (1H, dd, J=6.4, 4.9Hz, H5),
4.18 (1H, dd, J=6.4, 10.9Hz, H6),
4.23 (1H, dd, J=4.9, 10.9Hz, H6'),
4.40 (1H, d, J=9.4Hz, H3),
4.45 (1H, d, J=8.4Hz, H1),
5.00 (1H, s, H4).

$^{13}$C-NMR (68MHz) spectrum ($CD_3OD$ - $D_2O$) δ ppm :
14.7 ($CH_3$),
23.6, 26.7, 27.0, 30.2, 30.5, 30.7, 30.9, 32.9 ($CH_2$),
38.6 (CH),
67.3 (C6),
70.0 (C2),
72.5 (C5),
75.1 ($O\underline{C}H_2$),
75.8 (C4),
78.6 (C3),
104.1 (C1),

Compound 33 - 2

Mass spectrum ($FAB^-$) m/z :
985.3 $(M-Na)^-$.

$^1$H-NMR (270MHz) spectrum ($CD_3OD$ - $D_2O$) δ ppm :
0.88 (6H, t, J=6.8Hz, $CH_3$),
1.28 (52H, s, $C\underline{H}_2$),
1.6 - 1.7 (1H, m, $(C_{14}H_{29})_2C\underline{H}$),
3.48 (1H, dd, J=5.9, 9.3Hz, $(C_{14}H_{29})_2CHC\underline{H}aHb$),
3.80 (1H, dd, J=6.4, 9.3Hz, $(C_{14}H_{29})_2CHCHa\underline{H}b$),
4.10 (1H, dd, J=6.9, 5.4Hz, H5),
4.20 (1H, dd, J=6.9, 9.9Hz, H6),
4.25 (1H, dd, J=5.4, 9.9Hz, H6').
4.40 (1H, dd, J=7.4, 9.4Hz, H2),
4.55 (1H, dd, J=2.0, 9.4Hz, H3),
4.60 (1H, d, J=7.4Hz, H1),
5.10 (1H, d, J=2.0Hz, H4).

$^{13}$C-NMR (68MHz) spectrum ($CD_3OD$ - $D_2O$) δ ppm :
14.7 ($CH_3$),
23.6, 26.8, 27.0, 30.2, 30.4, 30.6, 30.8, 32.9 ($CH_2$),

38.5 (CH),
67.4 (C6),
72.6 (C5),
75.3 ($O\underline{C}H_2$),
76.1 (C4),
76.3 (C2),
76.9 (C3),
102.6 (C1).

Pharmacological Test Example

Anti-inflammatory action to lung damage model induced by CVF

The compounds obtained in the Examples and known compound (sulfatide) were selected as Test Compounds to check the inflammatory action of these compounds in the lung damage model induced by cobra venom factor (CVF), in accordance with the method described by M. S. Mulligan et al ["J. IMMUNOL.", Vol.151, page 6410 (1993); and "INT. IMMUNOL.", Vol.7, page 1107 (1995)].

Namely, the test compound was intravenously injected in amount of 0.2mg/rat to test animals (Lewis rats, age of 8 weeks, 5 animals in each group). 5 Minutes after administration, CVF (20U/2ml/kg) was administered to the tail vein, and after 30 minutes, a laparotomy was carried out under nembutal anesthesia to perfuse the lung with phosphate-buffered saline (PBS, pH7.4) by injecting the same from the right ventricle and to exenterate the lung. The extracted lung was homogenized with phosphate buffer (pH 7.4), and centrifuged to collect a supernatant. By spectroscopic analysis the index of bleeding was determined. (By measuring the amount of hemoglobin and thus detection wave-length was set to 541nm). Results are shown in following Table 1.

On the other hand, the precipitation obtained by centrifugation was sonicated in the presence of cetrimide and centrifuged to obtain a supernatant, the activity of myeloperoxidase therein being measured as an index of infiltration in neutrocyte. Results are shown in following Table 2.

Each inhibition rate (%) was calculated by 100 × [1 - (value of test compound - value of negative control)/(valueof positive control - value of negative control)].

Therefrom, it has been found that the compounds according to the invention show the anti-inflammatory action excellent than that of the sulfatide as exemplar known compounds.

Table 1

| Compound | Inhibition rate (%) |
|---|---|
| Example | |
| 1 - 1 | 22 |
| 1 - 2 | 16 |
| 2 | 36 |
| 3 - 1 | 27 |
| 3 - 2 | 49 |
| 4 | 39 |
| 5 | 52 |
| 6 | 0 |
| 7 - 1 | 36 |
| 7 - 2 | 42 |
| 8 | 53 |
| 9 - 1 | 84 |
| 9 - 2 | 100 |
| 10 - 1 | 65 |
| 10 - 2 | 63 |
| 11 - 1 | 100 |
| 11 - 2 | 82 |
| 12 | 0 |
| 13 | 43 |
| 14 | 55 |
| 15 | 45 |
| 16 | 75 |
| 17 | 74 |
| 18 | 50 |
| 19 | 43 |
| 20 | 45 |
| 21 | 64 |
| 22 | 65 |
| 23 - 1 | 53 |
| 23 - 2 | 51 |
| 24 - 1 | 54 |
| 24 - 2 | 50 |
| 25 | 56 |

| | |
|---|---|
| 26 | 56 |
| 27 - 1 | 65 |
| 27 - 2 | 62 |
| 28 - 1 | 63 |
| 28 - 2 | 63 |
| 29 | 67 |
| 30 | 70 |
| Sulfatide | 53 |

Table 2

| Compound | Inhibition rate (%) |
|---|---|
| Example | |
| 1 - 1 | 22 |
| 1 - 2 | 22 |
| 2 | 32 |
| 3 - 1 | 36 |
| 3 - 2 | 24 |
| 4 | 58 |
| 5 | 47 |
| 6 | 12 |
| 7 - 1 | 26 |
| 7 - 2 | 42 |
| 8 | 51 |
| 9 - 1 | 58 |
| 9 - 2 | 86 |
| 10 - 1 | 66 |
| 10 - 2 | 82 |
| 11 - 1 | 82 |
| 11 - 2 | 70 |
| 12 | 30 |
| 13 | 46 |
| 14 | 41 |
| 15 | 44 |
| 16 | 70 |
| 17 | 73 |

| | |
|---|---|
| 18 | 51 |
| 19 | 39 |
| 20 | 40 |
| 21 | 52 |
| 22 | 58 |
| 23 - 1 | 51 |
| 23 - 2 | 49 |
| 24 - 1 | 47 |
| 24 - 2 | 49 |
| 25 | 50 |
| 26 | 50 |
| 27 - 1 | 61 |
| 27 - 2 | 58 |
| 28 - 1 | 60 |
| 28 - 2 | 55 |
| 29 | 65 |
| 30 | 62 |
| Sulfatide | 50 |

Medicine Preparation Example 1 (Tablet)

Tablets were prepared in a conventional manner by using following ingredients.

| | |
|---|---|
| Compound (Example 16) | 2.0 (mg) |
| Lactose | 136.0 |
| Corn starch | 60.0 |
| Magnesium stearate | 2.0 |
| | 200.0 mg/tablet |

Medicine Preparation Example 2 (Injection)

A solution for injectional purpose was prepared in a conventional manner by using following ingredients and charged into ampules under aseptic condition to heat seal the ampules.

| | |
|---|---|
| Compound (Example 16) | 0.05 (mg) |
| Sodium chloride | 8.00 |
| Distilled water for injection | Remainder |
| | 1.0 ml/ampule |

## Claims

1. A sulfate or phosphate saccharide derivative of the formula

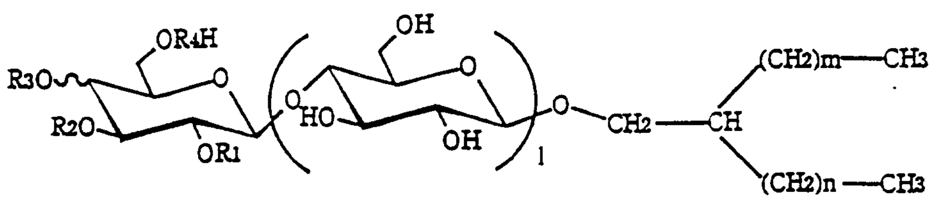

(I)

wherein $R_1$ is hydrogen atom or a residue of sulfate, phosphate or L-fucose; $R_2$, $R_3$ and $R_4$ are hydrogen atom or a residue of sulfate or phosphate, respectively; <u>l</u> is an integer of 0 or 1; m is an integer of 0-15 and <u>n</u> is an integer if 0-21,
or pharmaceutically acceptable salts thereof.

2. A sulfate or phosphate saccharide derivative or a salt thereof as claimed in Claim 1, wherein it is selected from the group consisting of

(1) 2-methylpropyl β-D-galactopyranoside 3-sulfate,
(2) 2-methylpropyl β-D-galactopyranoside 6-sulfate,
(3) 2-methylpropyl β-D-galactopyranoside 3,6-disulfate,
(4) 2-methylpropyl β-D-galactopyranoside 3,4,6-trisulfate,
(5) 2-methylpropyl β-D-galactopyranoside 2, 3, 4, 6-tetrasulfate,
(6) 2-propylpentyl β-D-galactopylanoside 3-sulfate,
(7) 2-propylpentyl β-D-galactopyranoside 6-sulfate,
(8) 2-propylpentyl β-D-galactopyranoside 3,6-disulfate,
(9) 2-propylpentyl β-D-galactopyranoside 2,3,6-trisulfate,
(10) 2-propylpentyl β-D-galactopyranoside 2,3,4,6-tetrasulfate,
(11) 2-hexadesyltetracosyl β-D-galactopyranoside 3-sulfate,
(12) 2-methylpropyl 0-β-D-galactopyranosyl-(1→4)-β-D-glucopyranoside 3'-sulfate,
(13) 2-methylpropyl 0-β-D-galactopyranosyl-(1→4)-β-D-glucopyranoside 3',6'-disulfate,
(14) 2-propylpentyl 0-β-D-galactopyranosyl-(1→4)-β-D-glucopyranoside 3'-sulfate.
(15) 2-propylpentyl 0-β-D-galactopyranosyl-(1→4)-β-D-glucopyranoside 3',6'-disulfate,
(16) 2-tetradesylhexadesyl 0-β-D-galactopyranosyl-(1→4)-β-D-glucopyranoside 3',6'-disulfate,
(17) 2-tetradesylhexadesyl 0-β-D-galactopyranosyl-(1→4)-β-D-glucopyranoside 3',6',6-trisulfate,
(18) 2-methylpropyl β-D-galactopyranoside 2,3,4,6-tetraphosphate,
(19) 2-propylpentyl β-D-galactopyranoside 2,3,4,6-tetraphosphate,
(20) 2-tetradesylhexadesyl β-D-galactopyranoside 2,3,4,6-tetraphosphate,
(21) 2-methylpropyl β-D-galactopyranoside 3,4-diphosphate,
(22) 2-propylpentyl β-D-galactopyranoside 3,4-diphosphate,
(23) 2-tetradesylhexadesyl β-D-galactopyranoside 3,4-diphosphate,
(24) 2-propylpentyl β-D-glucopyranoside 3-triphosphate,
(25) 2-propylpentyl β-D-glucopyranoside 2,3-diphosphate,
(26) 2-propylpentyl β-D-galactopyranoside 2,3,4-triphosphate,
(27) 2-propylpentyl 0-β-D-galactopyranosyl-(1→4)-β-D-glucopyranoside 3',4'-diphosphate,

(28) 2-tetradesylhexadesyl 0-β-D-galactopyranosyl-(1→4)-β-D-glucopyranoside 3',4'-diphosphate.
(29) 2-propylpentyl 0-α-L-fucopyranosyl-(1→2)-β-D-galactopyranoside 3-sulfate,
(30) 2-propylpentyl 0-α-L-fucopyranosyl-(1→2)-β-D-galactopyranoside 3,3'-disulfate.
(31) 2-propylpentyl 0-α-L-fucopyranosyl-(1→2)-β-D-galactopyranoside 3,6-disulfate,
(32) 2-propylpentyl 0-α-L-fucopyranosyl-(1→2)-β-D-galactopyranoside 3,3',6-trisulfate,
(33) 2-tetradesylhexadesyl 0-α-L-fucopyranosyl-(1→2)-β-D-galactopyranoside 3-sulfate,
(34) 2-tetradesylhexadesyl 0-α-L-fucopyranosyl-(1→2)-β-D-galactopyranoside 3,3'-disulfate,
(35) 2-tetradesylhexadesyl 0-α-L-fucopyranosyl-(1→2)-β-D-galactopyranoside 3,6-disulfate,
(36) 2-tetradesylhexadesyl 0-α-L-fucopyranosyl-(1→2)-β-D-galactopyranoside 3,3',6-trisulfate,
(37) 2-propylpentyl 0-α-L-fucopyranosyl-(1→2)-β-D-galactopyranoside 3-phosphate,
(38) 2-propylpentyl 0-α-L-fucopyranosyl-(1→2)-β-D-galactopyranoside 3,3'-diphosphate,
(39) 2-tetradesylhexadesyl 0-α-L-fucopyranosyl-(1→2)-β-D-galactopyranoside 3-phosphate,
(40) 2-tetradesylhexadesyl 0-α-L-fucopyranosyl-(1→2)-β-D-galactopyranoside 3,3'-diphosphate and
(41) a pharmaceutically acceptable salt of above compounds.

**3.** A process for the preparation of a sulfate or phosphate saccharide derivative of the Formula

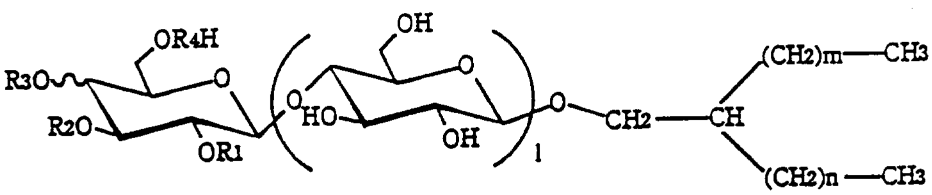

(I)

wherein $R_1$ is hydrogen atom or a residue of sulfate, phosphate or L-fucose; $R_2$, $R_3$ and $R_4$ are hydrogen atom or a residue of sulfate or phosphate, respectively; and l is an integer of 0 or 1; m is an integer of 0 - 15; n is an integer of 0 - 21,
or pharmaceutically acceptable salt thereof, which comprises a steps of reacting a compound of the Formula

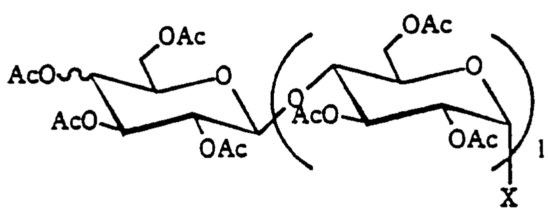

(II)

wherein l has the meaning as referred to and X is a removable group.
with a compound of the Formula

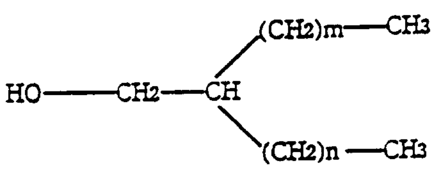

(III)

wherein m and n have the meanings as referred to,
and reacting the resulting saccharide derivative shown by a Formula

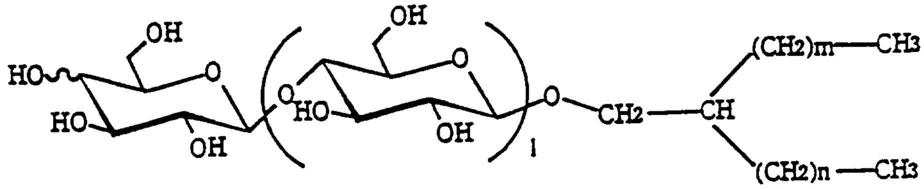

(IV)

wherein l, m and n have the meanings as referred to,
with di-n-butyl-tin oxide to make the saccharide derivative into its tin-acetal derivative, and then reacting the tin-acetal derivative with a complex of sulfite and trimethyl ammonium, in case of inserting a sulfate group in desired position; or reacting the saccharide derivative, if necessary, with 2,2-dimethoxy propane and then with a benzyl halide to protect a group in desired position in a conventional manner, removing the protection group, and then reacting with dibenzyloxi (diisopropylamino) phosphine and 1H-tetrazole, in case of inserting a phosphate group; and if necessary, converting the resulting sulfate or phosphate saccharide derivative into the salt.

**4.** A pharmaceutical composition which comprises

at least one sulfate or phosphate saccharide derivative or a pharmaceutically acceptable salt thereof, as claimed in Claim 1, in association with a pharmaceutically acceptable carrier or excipient.

**5.** Use of at least one sulphate or phosphate saccharide derivative as defined in anyone of claims 1-3 for the preparation of an anti-inflammatory agent.

European Patent Office

# EUROPEAN SEARCH REPORT

Application Number
EP 96 11 7513

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| D,X | BIOCHEMICAL AND BIOPHYSICAL RESEARCH COMMUNICATIONS, vol. 190, no. 2, 1993, ORLANDO, FL US, pages 426-434, XP000616343 Y.SUZUKI ET AL.: "Sulfated Glycolipids are Ligands for a Lymphocyte Homing Receptor, L-Selectin (LECAM-1), Binding Epitope in Sulfated Sugar Chain." * the whole document * | 1,5 | C07H3/06<br>C07H5/08<br>C07H11/04<br>A61K31/70 |
| D,A | INTERNATIONAL IMMUNOLOGY, vol. 7, no. 7, 1995, pages 1107-1113, XP000614962 M.S.MULLIGAN ET AL.: "Anti-Inflammatory Effects of Sulfatides in Selectin-Dependent Acute Lung Injury." * the whole document * | 1 | |
| A | US 4 694 076 A (OGAWA ET AL.) * abstract * | 1 | |
| A | WO 94 24167 A (W.REUTTER) * the whole document * | 1 | TECHNICAL FIELDS SEARCHED (Int.Cl.6)<br>C07H<br>A61K |
| P,A | EP 0 717 995 A (SANWA K.K. CO., LTD.) * the whole document * | 1 | |

The present search report has been drawn up for all claims

| Place of search | Date of completion of the search | Examiner |
|---|---|---|
| THE HAGUE | 27 January 1997 | Scott, J |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)